(19) Europäisches Patentamt
European Patent Office
Office européen des brevets

(11) Publication number: **0 358 047 A2**

# (12) EUROPEAN PATENT APPLICATION

(21) Application number: **89115558.2**

(22) Date of filing: **23.08.89**

(51) Int. Cl.5: **A01N 43/36 , A01N 47/40 , C07D 207/34 , C07D 207/42**

(30) Priority: **08.09.88 US 241648**
**11.08.89 US 391126**

(43) Date of publication of application:
**14.03.90 Bulletin 90/11**

(84) Designated Contracting States:
**AT BE CH DE ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Froyd, James Donald**
**313 Washington Avenue**
**Newtown Pennsylvania 18940(US)**
Inventor: **Smith, Diane Beth**
**202 River Road**
**Washington Crossing,Pennsylvania(US)**

(74) Representative: **Wächtershäuser, Günter, Dr.**
**Tal 29**
**D-8000 München 2(DE)**

## (54) Method of controlling phytopathogenic fungi.

(57) This invention is directed to a method for the control of phytopathogenic fungi by contacting the same with a cyano- or nitroarylpyrrole compound, or by applying said pyrrole compound to the foliage of a plant susceptible to disease caused by said pathogens.

EP 0 358 047 A2

# METHOD OF CONTROLLING PHYTOPATHOGENIC FUNGI

## BACKGROUND OF THE INVENTION

Phytopathogenic fungi are the causative agent for many diseases which infect and destroy agronomic crops. For example, fungi causes cucumber downy mildew, tomato late blight, rice blast, and wheat leaf rust as well as soilborne diseases such as root rot of wheat and damping off of cotton. To combat this problem, a variety of natural and synthetic fungicides have been utilized with varying degrees of success.

Even with the wide variety of the fungicides available today diseases caused by fungi still abound. Accordingly, there is an ongoing search in the art to create new and more effective methods of controlling plant pathogenic fungi.

## SUMMARY OF THE INVENTION

The present invention relates to a method of controlling plant pathogenic fungi, the causative agents for diseases infecting agronomic crops, both growing and harvested. The method includes applying certain cyano- or nitroarylpyrrole compounds directly to the fungi or to the foliage of the plant, the plant seed or to the soil surrounding the plant or plant seed. The compound can be applied in the form of a liquid spray (preferably aqueous), a fine ultrasonically prepared suspension, or as a dust or granular formulation. The fungicides of the present invention are especially useful and effective agents against the fungi which cause apple scab, cucumber downy mildew, grape downy mildew, tomato blight, rice blast, wheat leaf rust and wheat powdery mildew. They are also useful for the control of severe soilborne diseases such as root rot of wheat and damping off of cotton.

## DESCRIPTION OF THE INVENTION

The antifungal arylpyrrole compounds of the present invention have the structural formula illustrated below as formula I:

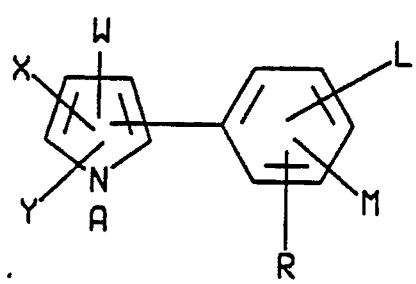

wherein X is H, F, Cl, Br, I or $CF_3$; Y is H, F, Cl, Br, I, $CF_3$ or CN; W is CN or $NO_2$ and A is H; $C_1$-$C_4$ alkyl optionally substituted with from one to three halogen atoms, one hydroxy, one $C_1$-$C_4$ alkoxy or one $C_1$-$C_4$ alkylthio, one phenyl optionally substituted with $C_1$-$C_3$ alkyl or $C_1$-$C_3$ alkoxy or with one to three halogen atoms, one phenoxy optionally substituted with one to three halogen atoms or one benzyloxy optionally substituted with one halogen substitutent; $C_1$-$C_4$ carbalkoxymethyl; $C_3$-$C_4$ alkenyl optionally substituted with from one to three halogen atoms; cyano; $C_3$-$C_4$ alkynyl optionally substituted with one halogen atom; di-($C_1$-$C_4$ alkyl) aminocarbonyl; or $C_4$-$C_6$ cycloalkylaminocarbonyl; L is H, F, Cl or Br; and M and R are each independently H, $C_1$-$C_3$ alkyl, $C_1$-$C_3$ alkoxy, $C_1$-$C_3$ alkythio, $C_1$-$C_3$ alkysulfinyl, $C_1$-$C_3$ alkysulfonyl, cyano, F, Cl, Br, I, nitro, $CF_3$, $R_1CF_2Z$, $R_2CO$ or $NR_3R_4$, and when M and R are on adjacent positions then with the carbon atoms to which they are attached they may form a ring in which MR represents the structure:

$-OCH_2O-$, $-OCF_2O-$ or 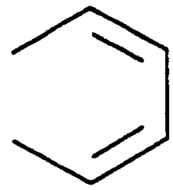 ;

Z is S(O)n or O; $R_1$ is H, F, $CHF_2$, CHFCl, or $CF_3$; $R_2$ is $C_1$-$C_3$ alkyl, $C_1$-$C_3$ alkoxy, or $NR_3R_4$; $R_3$ is H or $C_1$-$C_3$ alkyl; $R_4$ is H, $C_1$-$C_3$ alkyl, or $R_5CO$; $R_5$ is H or $C_1$-$C_3$ alkyl; and n is an integer of 0, 1 or 2, provided that when either X or Y or both are H, then

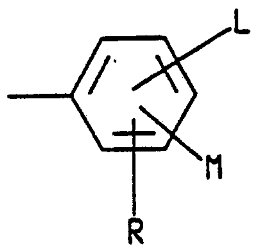

must be fixed to one of the two positions on the pyrrole ring adjacent to the nitrogen atom; provided also that when W is cyano and X and Y are both H, then at least one of L, M, and R is other than H; and further provided that when W is $NO_2$, A is $C_1$-$C_4$ alkyl, and either X or Y is Cl, then neither M nor R is $C_1$-$C_3$ alkoxy.

A preferred group of arylpyrroles are 2-arylpyrroles having the following formula:

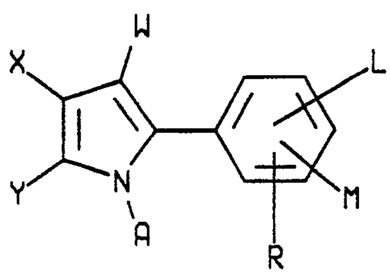

wherein A, L, M, R, W, X and Y are as described above.

Another preferred group of arylpyrroles of the present invention are those having the formula I structure: wherein A and W are as described above; X and Y are each Cl or Br; R is F, Cl, Br, $CF_3$, $NO_2$, or $OCF_3$; and M is H, F, Cl, or Br; and L is H, Cl, or Br.

Other arylpyrroles of this invention are the formula I compounds wherein W is $NO_2$; X and Y are hydrogen and L, M, R and A are as described above. These compounds find utility as intermediates for the preparation of the fungicidally active formula I arylpyrroles in which W is $NO_2$ and one or more of X, Y, L, M, R and A represents a substituent other than hydrogen.

The 2-arylpyrrole compounds wherein A is hydrogen; W is CN and X, Y, L, M and R are as described above, can be prepared by reacting N-formyl-DL-phenyl-glycine or a substituted N-formylphenylglycine represented by the structure formula II:

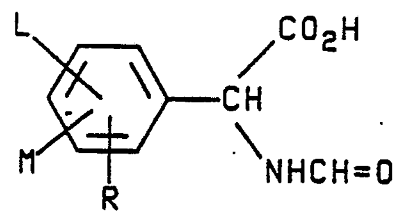

(II)

(wherein L, M and R are as described above) with at least an equivalent amount of a 2-chloroacrylonitrile and two to three equivalents of acetic anhydride. The reaction is conducted at an elevated temperature, preferably about 70°C to 100°C.

The reaction can be illustrated as follows:

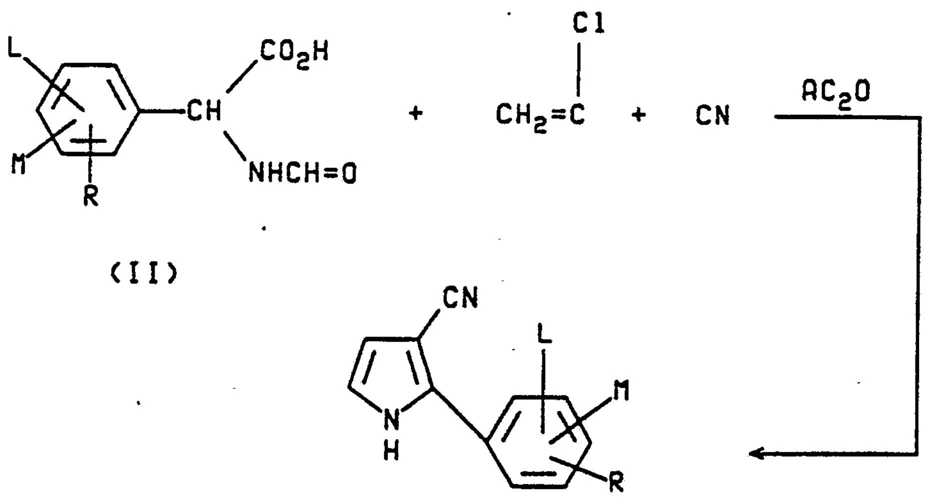


(II)

Conversion of the thus prepared 2-phenylpyrrole-3-carbonitrile or 2-(substituted phenyl)pyrrole-3-carbonitrile to the corresponding formula I, 4-halo, 5-halo or 4,5-dihalo-2-(substituted or unsubstituted phenyl)-pyrrole-3-carbonitrile, is readily achieved by reaction of said 2-phenylpyrrole-3-carbonitrile or 2-(substituted phenyl)pyrrole-3-carbonitrile with at least about 1 or 2 equivalents of a sulfuryl halide, bromine or chlorine, in the presence of a solvent such as dioxane, THF, acetic acid or a chlorinated hydrocarbon solvent. For preparation of a monohalo pyrrole-3-carbonitrile use of about 1 equivalent of the halogenating agent is required; whereas, preparation of a dihalo or trihalo pyrrole-3-carbonitrile requires 2 or 3 equivalents of said halogenating agent. When sulfuryl chloride or sulfuryl bromide is used the reaction is generally conducted at a temperature below about 40°C and preferably between about 0° and 30°C, but when elemental bromine is employed, the reaction is usually conducted at about 30-40°C. Other effective halogenating agents that may be employed in these reactions include sodium hypochlorite, t-butylhypochlorite, N-bromosuccinimide, and the like. The reaction may be illustrated as follows:

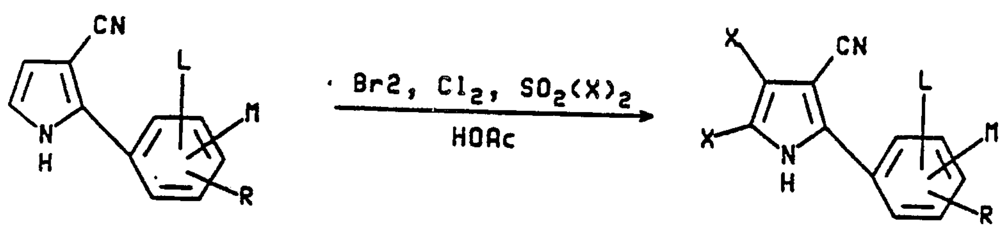


The formula I carbonitrile compounds of the present invention may also be prepared from the reaction of a substituted or unsubstituted benzoyl acetonitrile with a 2,2-di($C_1$-$C_4$ alkoxy)ethylamine in the presence of an aromatic solvent to form the α-(2,2-di($C_1$-$C_4$ alkoxy)ethylamino)-β-cyano-(substituted)styrene which is

then converted to the 2-(substituted-phenyl)pyrrole-3-carbonitrile of formula I by reaction of said $\beta$-3-cyano-(substituted)styrene compound with trifluoroacetic acid or concentrated HCl at a temperature between about 20° and 40°C. The reactions may be graphically illustrated as follows:

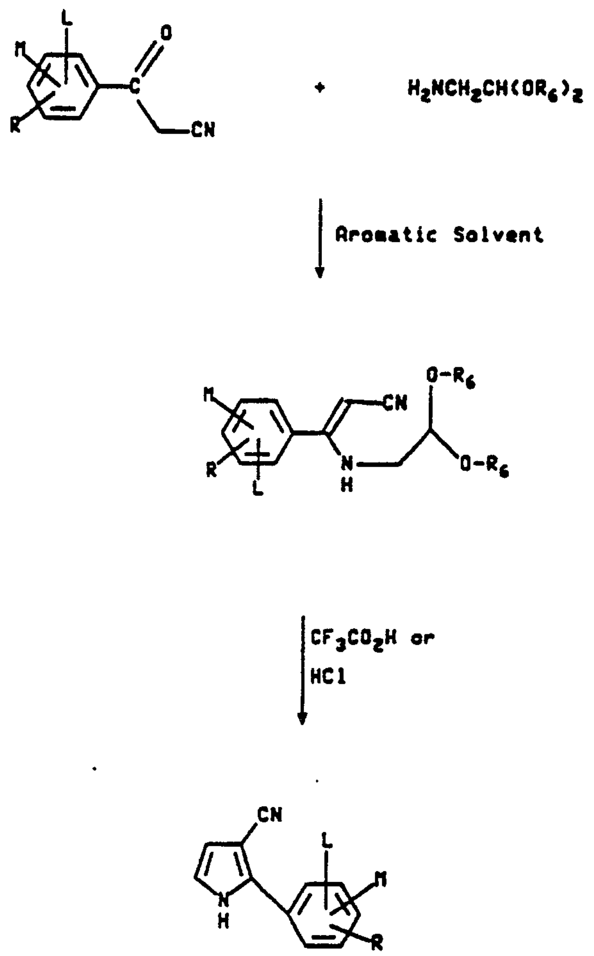


wherein $R_6$ is $C_1$-$C_4$ alkyl and L, R and M are as described above.

The formula (I) 3-nitro-2-phenylpyrrole and 3-nitro-2-(substituted)phenylpyrrole compounds can be prepared by reaction of an $\alpha$-nitroacetophenone or a substituted $\alpha$- nitroacetophenone with a 2,2-di($C_1$-$C_4$-alkoxy)ethylamine. The reaction is generally conducted in the presence of an inert organic solvent, preferably an aromatic solvent, at an elevated temperature to give an $\alpha$-(2,2-di($C_1$-$C_4$-alkoxy)ethylamino)-$\beta$-nitrostyrene or a substituted $\alpha$-(2,2-di($C_1$-$C_4$-alkoxy)ethylamino)-$\beta$-nitrostyrene that is converted to the formula (I) 3-nitro-2-phenylpyrrole or 3-nitro-2-(substituted)phenylpyrrole by treatment with a mineral acid such as hydrochloric or hydrobromic acid. Reaction of the thus prepared nitrophenylpyrrole with sodium hypochlorite in the presence of an inert organic solvent at a reduced temperature yields the formula (I) 2,3-dichloro-4-nitro-5-phenyl or 5-(substituted)phenylpyrrole.

The aforementioned reactions may be graphically illustrated as follows:

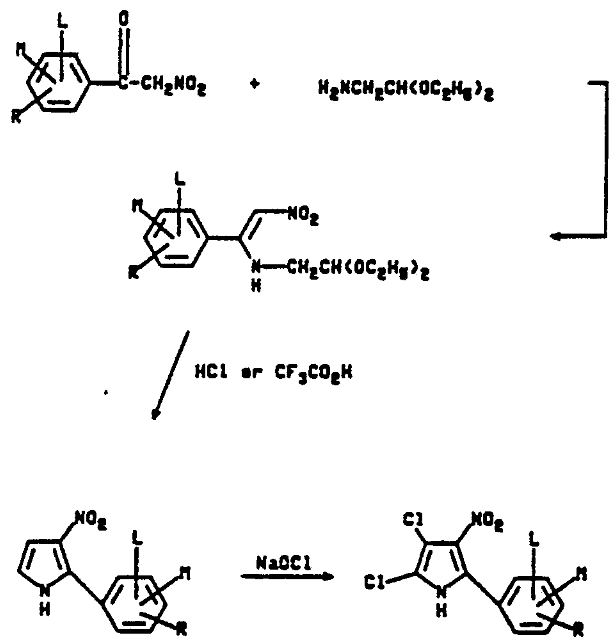


(I)

In addition to the several methods described in the literature for preparing substituted and unsubstituted benzoyl acetonitriles, surprisingly we have found that these compounds may also be prepared by reacting an appropriately substituted benzoyl halide with an alkali metal hydride and an alkyl cyanoacetate, such as t-butyl cyanoacetate, to yield the corresponding t-butyl(benzyl or substituted benzyl)cyanoacetate. These reactions may be graphically illustrated as follows:

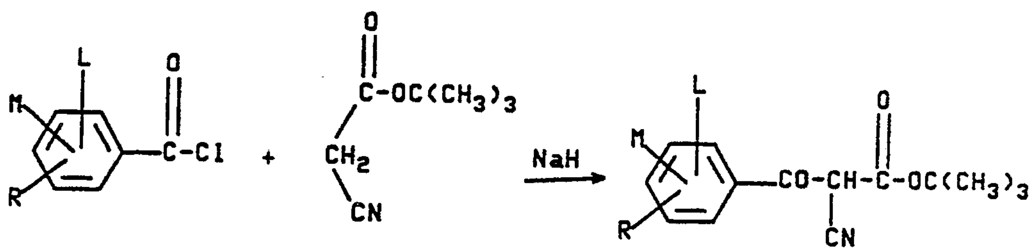


The thus formed cyanoacetate ester can then be converted to a substituted or unsubstituted benzoyl acetonitrile by heating the compound in toluene containing p-toluene sulfonic acid. The reaction may be graphically illustrated as follows:

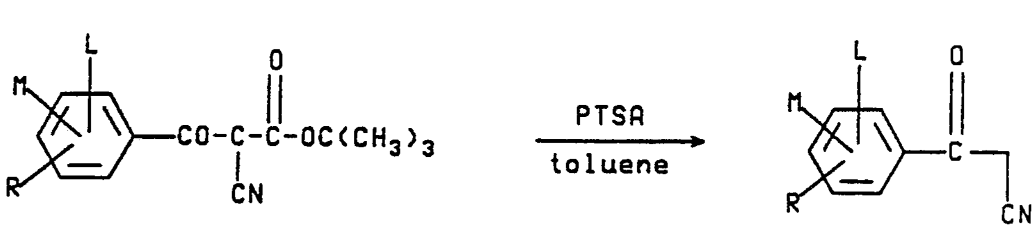


Examples of the t-butyl(benzoyl and substituted benzoyl)cyanoacetates and the reactions are shown in Table I and Table II.

**TABLE I**

**t-Butyl(benzoyl and Substituted benzoyl)cyanoacetates**

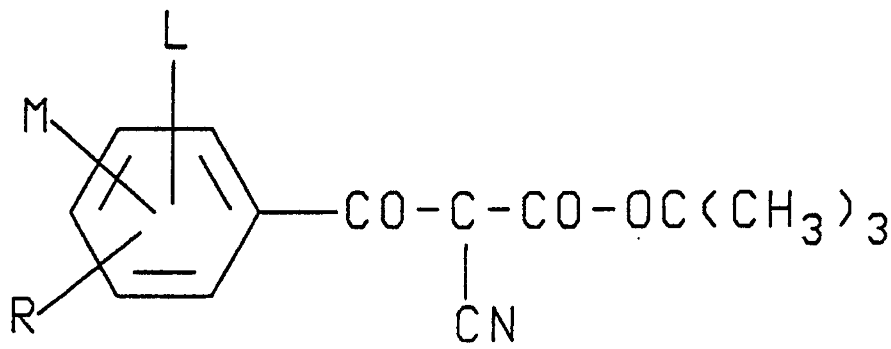

| L | M | R | mp°C |
|---|---|---|---|
| H | 3-Cl | 4-Cl | 91-94 |
| H | H | 4-$OCF_3$ | 81-84 |
| H | H | 4-Br | 113-115 |
| H | H | 4-$CF_3$ | 146-147 |
| H | H | 4-F | 98-100 |
| H | H | 4-CN | 127-128 |
| H | H | 4-$CF_3CH_2O$ | 136-139 |
| H | H | 4-$CH_3SO_2$ | 127-129 |
| H | 3-F | 4-F | 91-94 |
| H | H | 4-$CH_3S$ | 117-119.5 |
| H | H | 4-$CHF_2CF_2O$ | 92-94 |
| 3-Cl | 5-Cl | 4-$CH_3O$ | --- |

## TABLE II

## Benzoyl Acetonitriles

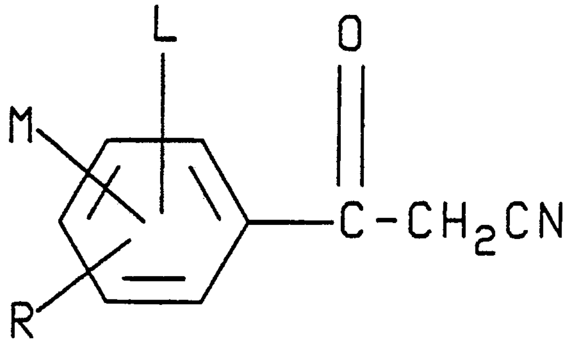

| L | M | R | mp°C |
|---|---|---|---|
| H | H | 4-Cl | 128.5-129.5 |
| H | 3-Cl | 4-Cl | 105-107 |
| H | H | 2-Cl | 53-55 |
| H | H | $4\text{-}OCF_3$ | 79-81 |
| H | H | $4\text{-}CF_3$ | 44-45 |
| H | 2-Cl | 4-Cl | 66-67 |
| H | H | 3-Cl | 80-83 |
| H | H | 4-CN | 126-128 |
| H | H | 4-F | 78-80 |
| H | H | $4\text{-}SO_2CH_3$ | 129-132 |
| H | 3-F | 4-F | 74-75 |
| H | H | $3\text{-}CF_3$ | 58-60 |
| H | H | $4\text{-}CH_3$ | 103.5-106 |
| H | H | $4\text{-}NO_2$ | 119-124 |
| 3-Cl | 5-Cl | $4\text{-}OCH_3$ | --- |

Preparation of N-substituted formula I arylpyrroles can be achieved by reaction of the appropriately substituted formula I arylpyrrole, wherein A is hydrogen and L, M, R, W, X and Y are as described above, with an appropriate alkylating agent and a suitable base, for example, a chloromethyl $C_1$-$C_4$ alkyl ether and potassium t-butoxide. This reaction provides an arylpyrrole having the same substituents as the starting material, but in addition is substituted on the nitrogen with $C_1$-$C_4$ alkoxymethyl. In a similar reaction cyanogen bromide is substituted for the brominated hydroxy $C_1$-$C_4$ alkyl and yields the formula I arylpyrrole with a carbonitrile substituent on the nitrogen. The reactions may be illustrated as follows:

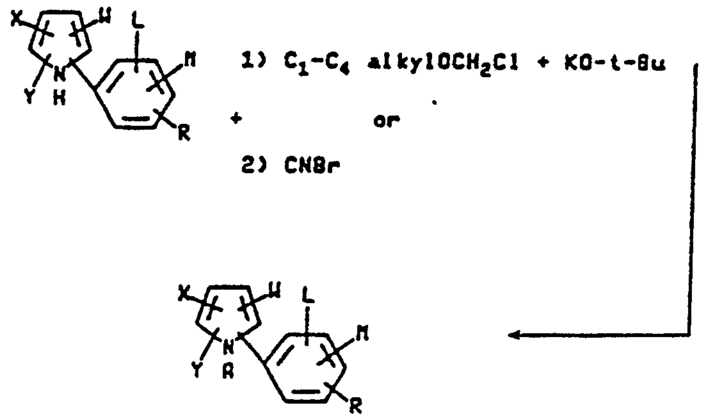


wherein L, M, R, W, X and Y are as described for formula I above and A is 1) $C_1$-$C_4$ alkoxymethyl or 2) CN.

Preparation of 2-phenylpyrrole 3,4-dicarbonitrile, 2-bromo-5-phenylpyrrole-3,4-dicarbonitrile and substituted phenyl derivatives thereof can be obtained by reaction of fumaronitrile with bromine in the presence of a chlorinated hydrocarbon such as chloroform at an elevated temperature to yield bromofumaronitrile. The thus formed bromofumaronitrile is then reacted with N-(trimethylsilyl)methyl-5-methyl-benzene-thioimidate or a substituted derivative thereof, in the presence of hexamethylphosphoramide at an elevated temperature to yield the 2-phenylpyrrole-3,4-dicarbonitrile. Bromination of the thus prepared 3,4-dicarbonitrile yields the 2-bromo-5-phenylpyrrole-3,4-dicarbonitrile or the substituted phenyl derivative if the substituted N-(trimethylsilyl)methyl-5-methyl-benzene-thioimidate is used in the previous reaction. The reaction may be graphically illustated as follows:

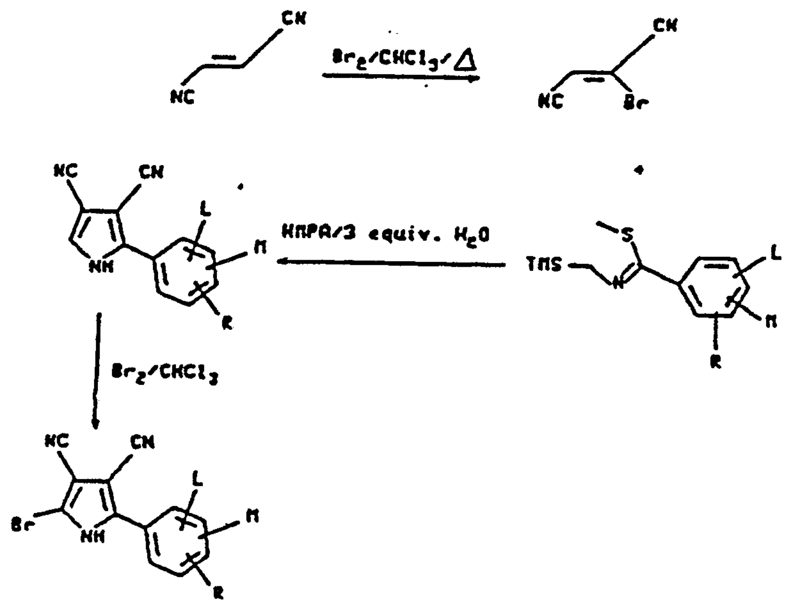


The examples provided by way of illustration below utilize the schemes illustrated above and provide a means for preparing other compounds of the invention which are not specifically described herein.

The arylpyrroles of the present invention, as hereinabove defined, are effective for controlling pathogenic fungi which are the causative agents for diseases which infect growing and harvested crops. Formula I arylpyrroles of this invention must be used in effective amounts. This will vary somewhat with the virility of the fungus in question and with other factors such as the environment in which treatment is conducted. Said compounds are especially useful and effective for the control of fungi which are the causative agents for cucumber downy mildew, tomato late blight, rice blast, and wheat leaf rust.

To protect plants from pathogenic fungi, the arylpyrroles of formula I are applied to the foliage of the plant, to the seed of the plants, or to the soil in which the plant grows or is to be grown, in the form of a liquid, preferably an aqueous spray, or dust, or granular formulation. Solutions or suspensions containing

from about 20 ppm to about 1,000 ppm, and preferably 50 ppm to 500 ppm, of formula I arylpyrroles are generally highly effective for this use.

The compounds of the invention can be formulated as emulsifiable concentrates, flowable concentrates, or wettable powders which are diluted with water or other suitable polar solvent, generally in situ, and then applied as a dilute spray. Said compounds may also be formulated in dry compacted granules, granular formulations, dusts, dust concentrates, suspension concentrates, microemulsions and the like all of which lend themselves to seed, soil, water and/or foliage applications to provide the requisite plant protection. Such formulations include the compounds of the invention admixed with inert, pharmacologically acceptable solid or liquid diluents.

For example, wettable powders, dusts, and dust concentrate formulations can be prepared by grinding and blending together about 25% to about 85% by weight of formula I arylpyrroles and about 75% to about 15% by weight of a solid diluent such as bentonite, diatomaceous earth, kaolin, attapulgite, or the like, 1% to 5% by weight of a dispersing agent such as sodium lignosulfonate, and about 1% to 5% by weight of a nonionic surfactant, such as octylphenoxy polyethoxy ethanol, nonylphenoxy polyethoxy ethanol or the like.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethyleneglycol, and 54% by weight of water.

A typical emulsifiable concentrate can be prepared by dissolving about 15% to 70% by weight of the active ingredient in about 85% to 30% by weight of a solvent such as isophorone, toluene, butyl cellosolve, methyl acetate, propylene glycol monomethyl ether, or the like and dispersing therein about 1% to 5% by weight of a nonionic surfactant such as an alkylphenoxy polyethoxy alcohol.

Application of the material is made by adding a predetermined quantity of formulated product, such as described above, to the desired volume of water or other suitable solvent, alone or in combination with other agronomic chemicals for simultaneous use.

In order to facilitate a further understanding of the invention, the following examples are presented primarily for the purpose of illustrating certain more specific details thereof. The invention is not to be deemed limited thereby except as defined in the claims. Unless otherwise noted, all parts are by weight and all temperatures are expressed as degrees centigrade.

## EXAMPLE 1

2-Phenylpyrrole-3-carbonitrile

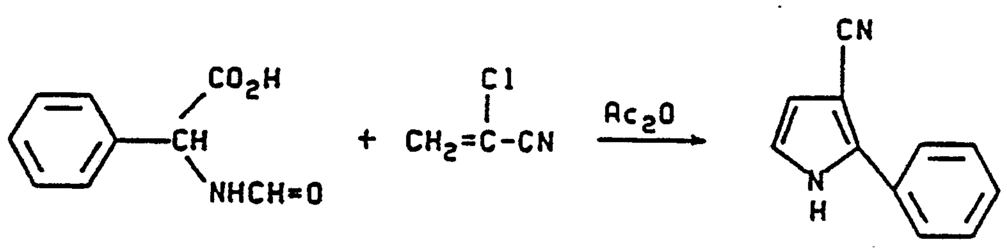


The following procedure is similar to the method given in JOC, 43, 4273-6 (1978). A magnetically stirred mixture of 30.00g of N-formyl-phenylglycine is heated at 90°C for 1 & 1/2 hours. The clear yellow reaction solution is concentrated in vacuo to give 42.5g of an oily brownish orange semi-solid. Material partially purified by chromatography on silica gel is shown by the proton NMR spectrum to be a mixture of 73% 2-phenylpyrrole-3-carbonitrile and 27% 2-phenyl-3-cyano-5-methylpyrrole. Recrystallization once from chloroform and twice from 1,2-dichloroethane gives 1.69g of an off-white solid which proton NMR shows it to be 96% 2-phenylpyrrole-3-carbonitrile, mp 148-152°C.

| Microanalysis (MW 168.19): | | | |
|---|---|---|---|
| Calcd.: | C, 78.55%; | H, 4.79%; | N, 16.66% |
| Found: | C, 78.52%; | H, 4.73%; | N, 16.54% |

## EXAMPLE 2

4,5-Dichloro-2-phenylpyrrole-3-carbonitrile and 5-chloro-2-phenylpyrrole-3-carbonitrile

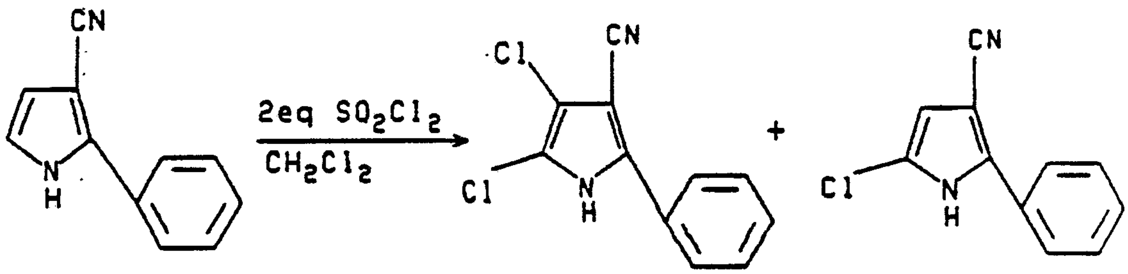


To a magnetically stirred ice-water cooled solution of 2.00g (11.9 mmol,) of 2-phenyl-3-cyano-pyrrole in 80 mL of methylene chloride is added dropwise over a period of 5 min., 1.90 mL (3.19 g, 23.6 mmol.) of sulfuryl chloride by means of a syringe. Throughout the addition the temperature is kept between 5°C and 10°C. Stirring at 5-10°C is continued for 90 minutes. The reaction mixture is vacuum filtered to remove a precipitated solid (1.28g) identified as 5-chloro-2-phenylpyrrole-3-carbonitrile, mp 192.5-195°C. The filtrate is diluted with 400 mL of ethyl acetate, washed twice with 200 mL of water, dried (sodium sulfate), treated with charcoal, filtered, and then concentrated in vacuo to give (after slurrying of the residue with hexane) 0.60g (21.3% yield) of a pink-purple solid. This solid is recrystallized from 5 mL of hot acetone to give 0.32g (9% yield) of 4,5-dichloro-2-phenylpyrrole-3-carbonitrile as an orangish brown solid, mp 254-255°C.
Max(mull,Nujol): 3165(br s), 3120(s), 2245(s), 1570(m), 1513(m), 1440(s), 1252(m), 1069(m), 996(m), 920-(m), 768(s), 698(s), 665(s) $cm^{-1}$.
H-NMR(DMSO): δ7.73 (d, J=6.6Hz, 1.97H, two phenyl protons at C-2,6), δ7.52 (t, J=7.3Hz, 2.04H, two phenyl protons at C-3,5), δ7.44 (t, J=7.3Hz, 1.02H, one phenyl proton at C-4).
C-NMR(DMSO): δ137.51 (C-2 pyrrole carbon), δ129.25 (C-4 phenyl carbon), δ129.04 (C-3,5 phenyl carbons), δ128.37 (C-1 phenyl carbon) δ125.88 (C-2,6 phenyl carbons), δ114.32 (either C-5 pyrrole or the nitrile carbon), δ114.14 (either C-5 pyrrole or the nitrile carbon), δ110.72 C-4 pyrrole carbon), δ89.78 (C-3 pyrrole carbon).

| Microanalysis (MW 237.09): | | | | |
|---|---|---|---|---|
| Calcd.: | C, 55.72%; | H, 2.55%; | N, 11.82%; | Cl, 29.91% |
| Found : | C, 55.78%; | H, 2.59%; | N, 11.12%; | Cl, 29.74% |

## EXAMPLE 3

p-Chloro-β-[(formylmethyl)amino]cinnamonitrile, diethyl acetal

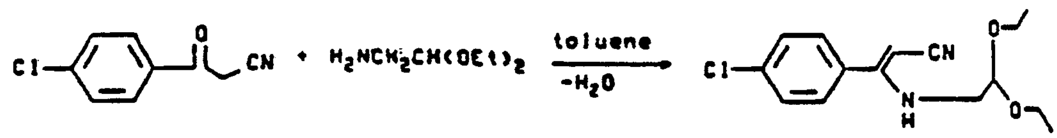


A magnetically stirred solution of 250.00 g (1.39 mol,) of p-chlorobenzoylacetonitrile, 203 mL (185.95 g, 1.39 mol) of 2,2-diethoxyethylamine, and 1300 mL of dried toluene is heated at refux for 20 hours. Water is collected in a Dean-Stark trap (23.8 mL, 95.2% theory). The hot cloudy dark brown solution with a large amount of undissolved solids is filtered through diatomaceous filter aid. After dilution with 200 mL of EtOAc, the solution is filtered through a 7cm X 13.5cm column of silica gel. The filtrate is concentrated in vacuo to give 354.38 g (86.4% crude yield) of a clear dark oil which slowly solidifies. This solid is recrystallized from hot cyclohexane to give 324.26g (79.1% yield) of a waxy orange solid. NMR of this product shows it to be composed of 78% (Z) and 23% (E) isomeric mixture of p-chloro-β-[(formylmethyl)amino] cinnamonitrile, diethyl acetal, m.p. 60-72°C. The following analytical data is for another similarly prepared sample.

Max(mull,Nujol): 3325(s), 3065(m), 2197(s), 1600(s), 1530(s), 1314(m), 1265(m), 1173(m), 1154(m), 1128(s), 1100(s), 1060(s), 1022(s), 939(m), 895(m), 844(s), 768(m), 730(m) $cm^{-1}$.

H-NMR(chloroform): δ7.47 (d, J=8.6Hz, 2.12H, two aromatic protons), δ7.37 (d, J=8.6Hz, 2.12H, two aromatic protons), δ5.10(E) & δ4.86(Z) [br t, 1.25H, one N-H proton], δ4.69(Z) & δ4.60(E) [t, J=5.1Hz, 1.05H, one methine proton at the acetal carbon], δ4.07 (E) & δ4.05(Z) [s, 0.83H, enamine β proton], δ3.71(E) & δ3.68(Z) [q, J=7.1Hz, 2.22H, two methylene protons of one of two ethoxy groups], δ3.56(Z) & δ3.53(E) [q, J=7.1Hz, 2.22H, two methylene protons of one of two ethoxy groups], δ3.18 (t, J=5.1Hz, 1.77H, two methylene protons of the ethyleneacetal group), δ1.20 (t, J=7.1Hz, 4.90H, six methyl protons of the two ethoxy groups).

C-NMR(chloroform): δ161.21 (α-enamine carbon), δ136.29 (Z) & δ134.60(E) [either C-1 or C-4 of the phenyl ring], δ134.08(Z) & δ132.30(E) [either C-1 or C-4 of the phenyl ring], δ129.34(Z) & δ129.89(E) [either C-2,6 or C-3,5 of the phenyl ring], δ128.94(Z) & δ128.63(E) [either C-2,6 or C-3,5 of the phenyl ring], δ121.19(Z) & δ119.50(E) [nitrile carbon], δ99.43(Z) & δ100.63(E) [β-enamine carbon], δ61.88(Z) & δ63.25(E) [methine carbon of the acetal], δ62.64(Z) & δ63.03(E) [methylene carbons of the ethoxy groups], δ46.32(Z) & δ47.33-(E) [methylene carbon of the ethyl amine group], δ15.26 (methyl carbons of the ethoxy groups).

| Microanalysis (MW 294.78): | | | | |
|---|---|---|---|---|
| Calcd:<br>Found: | C, 61.11%;<br>C, 61.25%; | H, 6.50%;<br>H, 6.25%; | N, 9.51%;<br>N, 9.34%; | Cl, 12.03%.<br>Cl, 12.35%. |

## EXAMPLE 4

2(p-Chlorophenyl)-pyrrole-3-carbonitrile

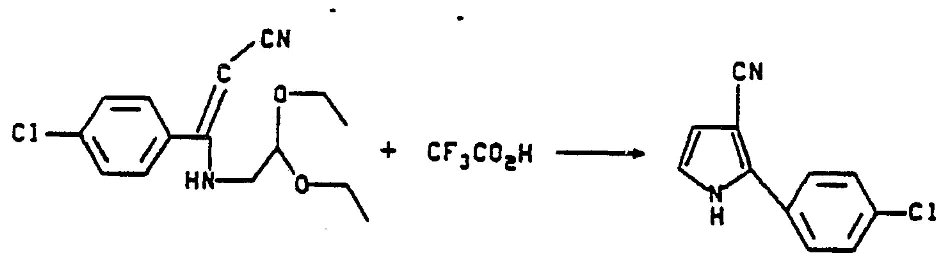

To 108 mL of trifluoroacetic acid stirred at 23°C is added 54.00 g (0.183 mol) of solid p-chloro-β-[-(formylmethyl)amino]cinnamonitrile, diethyl acetal over a period of 45 minutes. This addition produced an

exotherm to 38°C and, 32 minutes into the addition, a solid started to precipitate. After stirring at room temperature for 30 minutes, the reaction mixture is vacuum filtered and the collected solid is washed first with trifluoroacetic acid, secondly with an ethyl acetate-hexane mixture, and finally with hexane. The yield is 16.83 g (45.4%) of an off-white solid, mp 165-166°C. The following anal. data is from a similarly prepared sample.

Max(mull, Nujol): 3275(br s), 2225(s), 1502(s), 1410(m), 1275(m), 1200(m), 1108(s), 1023(m), 999(m), 908-(m), 843(s), 752(s), 722(s), 695(s), 620(s) $cm^{-1}$.

H-NMR(acetone): δ11.22 (v br s, 0.99H, one pyrrole N-H proton), δ7.82 (d, J=8.9Hz, 2.46H, two aromatic phenyl protons), δ7.51 (d, J=8.9Hz, 2.46Hz, two aromatic phenyl protons), δ7.02 (t, J=2.6Hz, 1.01H, one pyrrole proton at C-5), δ6.58 (t, J=2.6Hz, 0.77H, one pyrrole proton at C-4).

C-NMR(acetone): δ137.73 (pyrrole C-2), δ134.42 (p-chlorophenyl at C-4), δ129.93 (methine carbons at C-3,5 of the phenyl ring), δ128.07 (methine carbons at C-2,6 of the phenyl ring), δ121.21 (pyrrole at C-5), δ117.93 (nitrile carbon), δ113.78 (pyrrole carbon at C-4), δ90.86 (pyrrole carbon at C-3).

| Microanalysis (MW 202.64): | | | | |
|---|---|---|---|---|
| Calcd.: | C, 65.19%; | H, 3.48%; | N, 13.83%; | Cl, 17.50% |
| Found: | C, 64.18%; | H, 3.52%; | N, 13.63%; | Cl, 17.74% |

Use of the above procedure as shown or with the substitution of concentrated hydrochloric acid for trifluoroacetic acid affords the following compounds:

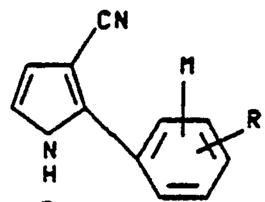

| M and/or R | mp°C | Acid Used |
|---|---|---|
| 4-Cl | 165-166 | conc. HCl, $CF_3COOH$ |
| 3,4-di-Cl | 216-221 | $CF_3COOH$ |
| 2-Cl | 156-157 | $CF_3COOH$ |
| 4-$OCF_3$ | 143-145 | $CF_3COOH$ |
| 4-$CF_3$ | 179-180 | $CF_3COOH$ |
| 2,4-di-Cl | 197-199 | $CF_3COOH$ |
| 3-Cl | 150-156 | $CF_3COOH$ |
| 4-CN | 210-212 | $CF_3COOH$ |
| 4-F | 167-170 | conc. HCl |
| 4-$SO_2CH_3$ | 221-221.5 | $CF_3COOH$ |
| 3,4-di-F | 173-175.5 | $CF_3COOH$ |
| 3-$CF_3$ | 166-168 | $CF_3COOH$ |
| 4-$COOCH_3$ | 155.5-158 | $CF_3COOH$ |
| 4-$CH_3$ | 117-137 | $CF_3COOH$ |
| 4-$NO_2$ | 174-177 | $CF_3COOH$ |

EXAMPLE 5

4,5-Dichloro-2-(p-chlorophenyl)pyrrole-3-carbonitrile

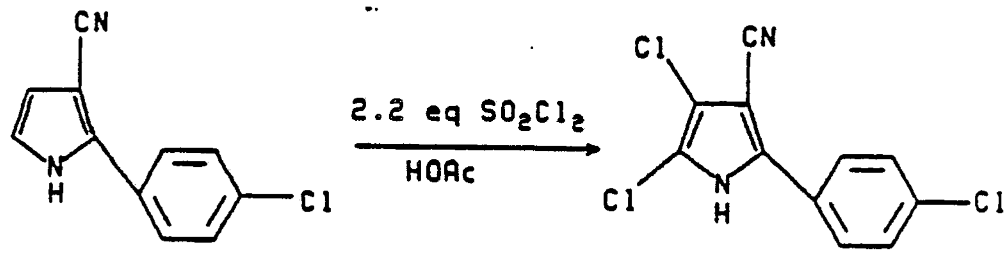


To a mechanically stirred solution of 16.83g (83.1 mmol) of 2-(p-chlorophenyl)pyrrole-3-carbonitrile in 450 mL of glacial acetic acid at 36°C is added dropwise 14.7 mL (24.70 g, 183.0 mmol) of sulfuryl chloride over a period of 18 minutes. The addition produces a slight exotherm to 39°C and, after another 16 minutes, the reaction mixture is vacuum filtered. The collected solids are washed first with acetic acid and then with water. This solid after recrystallization from hot ethyl acetate, melts at 259-261°C. By similar procedures other samples of this product were prepared and the analytical data for one such product is shown below.
Max(mull, Nujol): 3170(br s), 3100(m), 2225(s), 1508(m), 1097(m), 825(s), 717(m), 660(m) $cm^{-1}$.
H-NMR(DMSO): d7.72 (d, J=8.6Hz, 2.00H, two aromatic protons), δ7.56 (d, J=8.6Hz, 2.00H, two aromatic protons).
C-NMR(DMSO): δ136.01 (pyrrole C-2 carbon), δ133.92 (p-chlorophenyl C-4 carbon), δ129.09 (p-chlorophenyl C-3,5 carbons), δ127.41 (p-chlorophenyl C-4 carbon), δ127.11 (p-chlorophenyl C-1 carbon), δ114.49 (nitrile carbon), δ114.10 (pyrrole C-5 carbon), δ110.92 (pyrrole C-4 carbon), δ90.09 (pyrrole C-3 carbon).

| Microanalysis (MW 271.54): | | | | |
|---|---|---|---|---|
| Calcd.: | C, 48.65%, | H, 1.86%; | N, 10.32%; | Cl, 39.17% |
| Found: | C, 49.22%; | H, 2.12%; | N, 9.85%; | Cl, 39.03% |

EXAMPLE 6

4,5-Dibromo-2-(α,α,α-trifluoro-p-tolyl)-pyrrole-3-carbonitrile

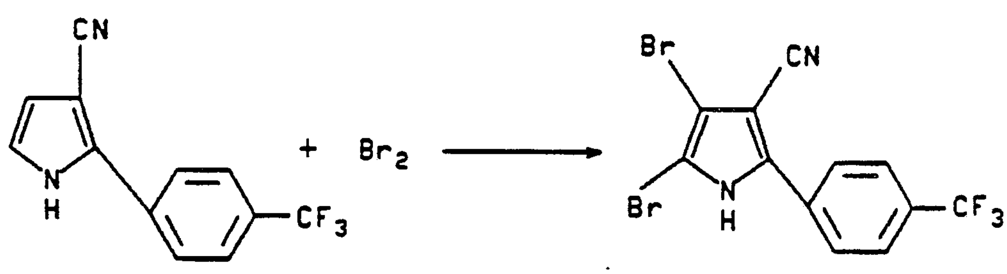

To a stirred mixture of 0.8g of 2-(α,α,α-trifluoro-p-tolyl)pyrrole-3-carbonitrile in 70 mL of chloroform is added 2 mL of bromine. The mixture, on stirring overnight, deposits a white solid which is collected by filtration. Thin layer chromatography (1:1 ethyl acetate-hexane) shows a single component; m.p. >230°C.

| Anal. Calc'd for $C_{12}H_5Br_2F_3N_2$; | C, 36.55; | H, 1.27; | N, 7.11; | Br, 40.61. |
|---|---|---|---|---|
| Found: | C, 36.40; | H, 1.08; | N, 6.99; | Br, 40.55. |

Following the procedures of Examples 5 and 6, but substituting the appropriately substituted phenylpyrrole-3-carbonitrile for 2-(α,α,α-trifluoro-p-tolyl) pyrrole-3-carbonitrile yields the following compounds.

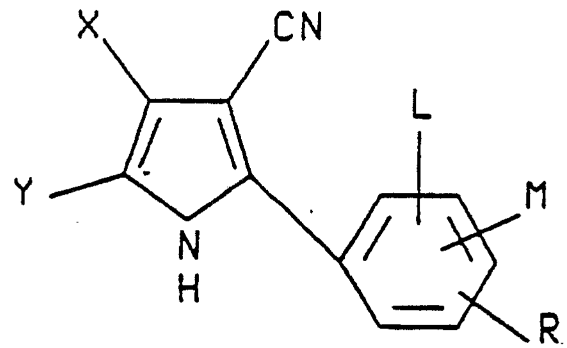

| L | M | R | X | Y | mp°C |
|---|---|---|---|---|---|
| H | H | 4-$NO_2$ | Br | Br | 274-277 |
| H | H | 4-F | Cl | Cl | >220 |
| H | H | 4-F | Br | Br | >220 |
| H | H | 4-$SO_2CH_3$ | Cl | Cl | >230 |
| H | 3-F | 4-F | Cl | Cl | >230 |
| H | 3-F | 4-F | Br | Br | >220 |
| 2-Cl | 3-Cl | 4-Cl | Cl | Cl | |
| 2-Br | 3-Br | 4-Br | Br | Br | |
| H | H | 4-$OCF_3$ | Cl | Cl | 222-225 |
| H | H | 4-$OCF_3$ | Br | Br | 231-232 |
| H | H | 4-$OCF_3$ | Cl | H | |
| H | H | 4-CN | Br | Br | >230 |
| H | H | 4-CN | Cl | Cl | >240 |
| H | H | 4-$SO_2CH_3$ | Br | Br | >230 |
| H | H | 4-$NO_2$ | Cl | Cl | 246-249 |
| H | 3-Cl | 4-Cl | Br | Br | >260 |
| H | H | 3-$CF_3$ | Cl | Cl | >230 |
| H | H | 4-$COCH_3$ | Cl | Cl | 251-254 |
| H | 2,3-CH=CH- | | Cl | Cl | 244-247 |
| H | H | 4-$CH_3$ | Cl | Cl | 215-217 |
| H | 2-Cl | 4-Cl | Br | Br | >230 |

| L | M | R | X | Y | mp°C |
|---|---|---|---|---|---|
| H | H | 3-Cl | Cl | Cl | >230 |
| H | 2-Cl | 4-Cl | Cl | Cl | >230 |
| H | H | 4-Cl | Br | Br | 273-274 |
| H | H | 2-Cl | Br | Br | >230 |
| H | H | $4-CF_3$ | Cl | Cl | >230 |
| H | H | 4-Br | Cl | Cl | >235 |
| H | H | 2-Cl | Cl | Cl | >230 |
| H | 3-Cl | 4-Cl | Cl | Cl | >235 |
| H | H | H | Cl | Cl | 254-255 |
| H | H | 4-Cl | Cl | Cl | 255-257 |
| H | H | $4-CF_3$ | Br | Br | >230 |
| H | H | 4-Cl | Cl | Br | 262-263(dec.) |
| H | H | 4-Cl | Br | Cl | 250-258(dec.) |
| H | 3-Cl | 5-Cl | Cl | Cl | >230 |
| H | 3-Cl | 4-Cl | Cl | Br | >230 |
| 2-Cl | 4-Cl | 5-F | Cl | Cl | 207-210 |

## EXAMPLE 7

3-Nitro-2-phenylpyrrole

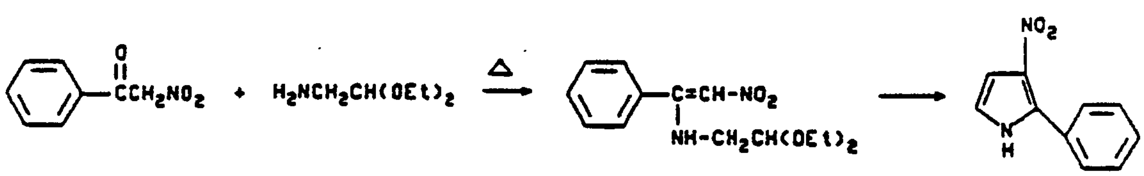

Alpha-nitro acetophenone (5.7 g, 0.0345mol) is taken up in 100 mL toluene and 4.6g (0.0345mol) of amino acetaldehyde diethyl acetal is added. The reactants are put into a 250 mL RB flask fitted with a Dean-Stark trap. The trap is filled with 4A molecular sieves and the mixture is heated at reflux for 18 hours. The toluene is removed in vacuo to give 8.36 g of α-(2,2-diethoxyethylamino)-β-nitrostyrene as a brown oil. To this oil is added 50 mL of concentrated HCl. As the flask is swirled the oil turns to a yellow suspension. After 10 minutes the solid is filtered to give 2.48 g of a yellow solid. Recrystallization from ether/ethylacetate/hexane gives the product as two fractions, 2.08 g of m.p. 190-192°C, (31%). Max 1485 $cm^{-1}(NO_2)$, H-NMR($CDCl_3$/DMSO) δ6.73(m,2H), 7.46(m.5H).

## EXAMPLE 8

2,3-Dichloro-4-nitro-5-phenylpyrrole

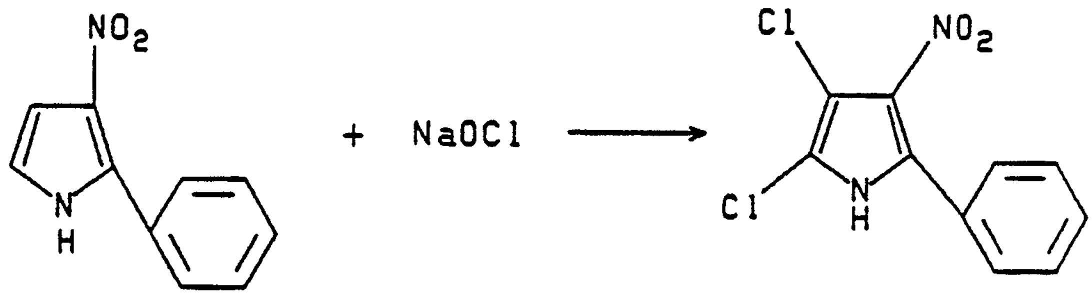

A mixture of 3-nitro-2-phenylpyrrole (1.56g, 0.0083mol) in 60 mL of dioxane is cooled in an ice bath while 25.9g (.0182mol) of commercial sodium hypochlorite is added dropwise. After stirring for 45 minutes, the mixture is acidified with concentrated HCl. Water and $Et_2O$ are added. The layers are separated and the top organic layer is washed with $H_2O$, dried over anhydrous $MgSO_4$ and concentrated in vacuo to give 2.21g of yellow solid. Purification by chromatography using silica gel and eluting with increasing ratios of ethyl acetate/ hexane gives, after stripping, 0.77g of yellow solid (36%) m.p. 190-190.5°C;

| | | | |
|---|---|---|---|
| Analysis: Calcd. for $C_{10}H_6N_2O_2Cl_2$ | C, 46.72; | H, 2.35; | N, 10.90 |
| Found: | C, 46.96; | H, 2.86; | N, 10.02 |

Following the procedures of Examples 7 and 8 above but using the appropriately substituted α-nitroacetophenone and 2,2-di($C_1$-$C_4$ alkoxy)ethylamine yields the substituted α-(2,2-di($C_1$-$C_4$ alkoxy)-ethylamino)-β-nitrostyrene which is then converted to 3-nitro-2-(substituted)phenylpyrrole by treatment with HCl, HBr or $CF_3CO_2H$. Reaction of the thus formed substituted phenylpyrrole with sodium hypochlorite in dioxane yields the chloro analogs; whereas, reaction of the substituted phenylpyrrole with bromine in chloroform yields the bromine analogs.

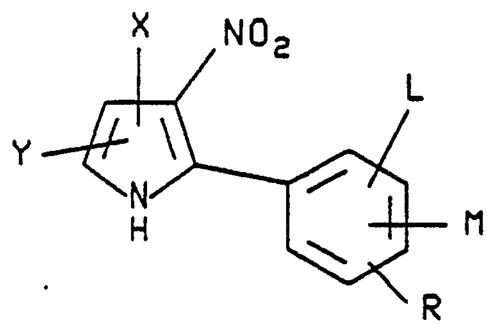

| L | M | R | X | Y | mp°C |
|---|---|---|---|---|---|
| H | H | H | Cl | Cl | 190-190.5 |
| H | 4-Cl | H | Cl | Cl | 214-215 |
| H | 4-Cl | H | Br | Br | 203-204(dec.) |
| H | H | H | Br | Br | 148.5-149 |
| 3-Cl | 4-Cl | C | Cl | Cl | 219-220(dec.) |
| H | 4-Br | H | Cl | Cl | 222-223(dec.) |
| H | H | 4-$CF_3$ | Cl | Cl | 166-168 |

EXAMPLE 9

4,5-Dichloro-2-(3,4-dichlorophenyl)-1-methyl-pyrrole-3-carbonitrile

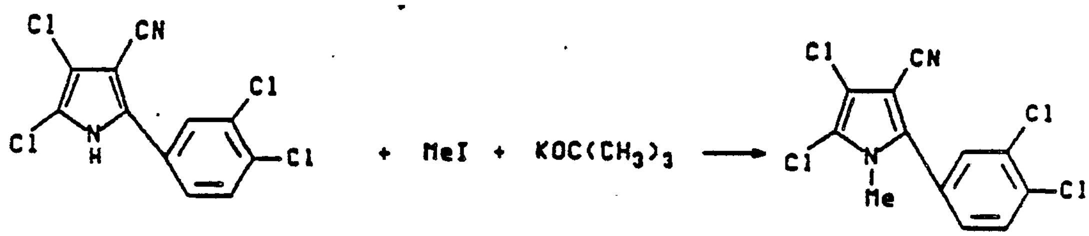


In a 100 mL flask, 2 g of 4,5-dichloro-2-(3, 4-dichlorophenyl)pyrrole-3-carbonitrile in 60 mL dry THF gives a clear brown solution. 1 eq of KOtBu is added w/ stirring, this giving a clear solution after a few minutes. 1 eq of MeI is added by syringe and the solution is heated at reflux for 4 hours. It is then left to stir at RT overnight. The following day 50 mL of $H_2O$ is added and the mixture extracted with 4 x 50 mL $CHCl_3$. The organic phases are combined, dried with $MgSO_4$, and concentrated. The resulting white solid is purified by flash chromatography on silica gel, using 50/50 EtOAc/hexane as an eluent. This gives 1.80 g of a white solid.

Yield = 86%

m.p. = 154-156 deg. C

Following the above procedure but substituting the appropriately substituted phenylpyrrole-3-carbonitrile or 3-nitro-2-(substituted)phenylpyrrole for 4,5-dichloro-2-(3,4-dichlorophenyl)pyrrole-3-carbonitrile yields the compounds shown below.

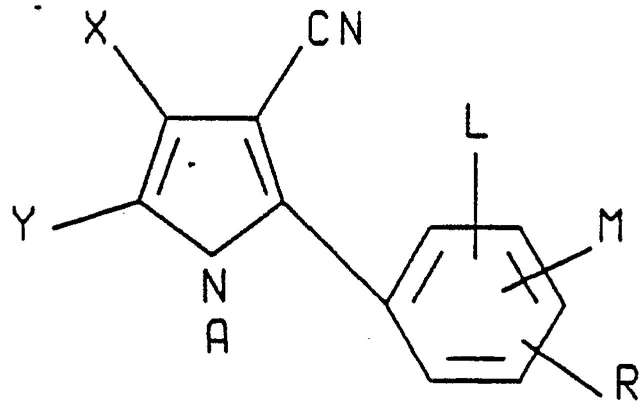

| A | L | M | R | X | Y | mp°C |
|---|---|---|---|---|---|---|
| $CH_3$ | H | H | 4-Cl | Cl | Cl | 152-153 |
| $C_2H_5OCH_2$ | H | 3-Cl | 4-Cl | Cl | Cl | 128-130 |
| $C_2H_5$ | H | 3-Cl | 4-Cl | Cl | Cl | 137-138 |
| $CH_3$ | H | 3-Cl | 4-Cl | Cl | Cl | 154-156 |
| $CH_3$ | H | H | $4-CF_3$ | Br | Br | 145-146 |
| $C_6H_5-CH_2$ | H | H | $4-CF_3$ | Br | Br | 145-147 |
| $C_6H_5-CH_2$ | H | 3-Cl | 4-Cl | Cl | Cl | 95-96 |
| $CH_2=CH-CH_2$ | H | 3-Cl | 4-Cl | Cl | Cl | 69-70 |
| $CH_2=C(Cl)-CH_2$ | H | 3-Cl | 4-Cl | Cl | Cl | |
| $CH\equiv C-CH_2$ | H | 3-Cl | 4-Cl | Cl | Cl | 147-148 |
| $CH_3SCH_2$ | H | 3-Cl | 4-Cl | Cl | Cl | |
| $C(CH_3)_3$ | H | H | $4-CF_3$ | Cl | Cl | |
| $CH_3$ | H | H | $4-CF_3$ | Cl | Cl | 99-100 |
| $CH_3SC_2H_5$ | H | 3-Cl | 4-Cl | Cl | Cl | 74-75 |
| $C_2H_5-OC(=O)-CH_2$ | H | 3-Cl | 4-Cl | Cl | Cl | 118-120 |
| $C_2H_5-OCH_2$ | H | H | $4-CF_3$ | Cl | Cl | 99-100 |
| $CH_3$ | H | H | $4-OCH_3$ | Br | Br | 112-115 |
| $CH_3$ | H | H | 4-Cl | Br | Br | 197-201 |
| $C_2H_5OCH_2$ | H | H | $4-OCF_3$ | Cl | Cl | 46-47 |
| $CH_3$ | H | H | $4-OCF_3$ | Cl | Cl | 72-73 |
| $C_6H_5-CH_2$ | H | H | $4-OCF_3$ | Cl | Cl | oil |
| $C_2H_5OCH_2$ | H | H | 4-Cl | Cl | Cl | - |

| A | L | M | R | X | Y | mp°C |
|---|---|---|---|---|---|---|
| $HOCH_2CH_2$ | H | 3-Cl | 4-Cl | Cl | Cl | 143-145 |
| NC | H | 3-Cl | 4-Cl | Cl | Cl | 251-252 |
| $C_6H_5CH_2OCH_2$ | H | 3-Cl | 4-Cl | Cl | Cl | 88-89 |
| Cl O-$CH_2$ | H | 3-Cl | 4-Cl | Cl | Cl | 118-120 |
| IC=C-$CH_2$ | H | 3-Cl | 4-Cl | Cl | Cl | 115-116 |
| $CH_3$ | H | H | 4-Cl | Br | $CF_3$ | 126-129 |
| $C_2H_5OCH_2$ | H | H | 4-Cl | Br | $CF_3$ | 91-92 |
| $C_2H_5$-$OCH_2$ | H | 3-Cl | 4-Cl | Cl | Cl | 118-120 |
| $C_2H_5$-$OCH_2$ | H | H | 4-Cl | Br | Br | 104-105 |
| $C_6H_5$-$CH_2$ | H | H | 4-Cl | Br | Br | 81-82 |
| $CH_3$ | H | H | 4-Cl | Br | Br | 197-201 |
| CN | H | H | 4-$CF_3$ | Cl | Cl | 138-139 |
| $C_2H_5$-$OCH_2$ | H | H | 4-$CF_3$ | Br | $CF_3$ | 104-105 |
| $C_2H_5$-$OCH_2$ | H | H | 4-$CF_3$ | H | $CF_3$ | 76-77 |
| $C_2H_5OCH_2$ | H | 3-Cl | 4-Cl | Br | $CF_3$ | 80-81 |

## EXAMPLE 10

1-Benzyl-4,5-dibromo-2-(α,α,α-trifluoro-p-tolyl) pyrrole-3-carbonitrile

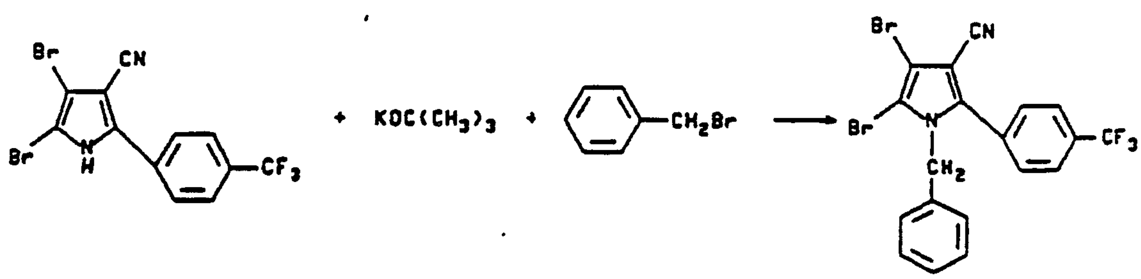


In a 100 mL flask, 1.5 g of 4,5-dibromo-2-(α,α,α-trifluoro-p-tolyl)pyrrole-3-carbonitrile is mixed with 50 mL dry THF to give a clear dark solution. 1 eq of KOtBu is added with stirring. After a few minutes the solution clears. Benzyl bromide (0.65 g) is added by syringe. The mixture is heated at reflux overnight. The following day TLC (50/50 EtOAc/hexane) indicates the presence of both starting material and product. The reaction is worked up in the following manner; 50 mL of water is added and the mixture is extracted with 4 x 50 mL $CHCl_3$. The organic phases are combined and washed with 4 x 50 mL 10% aq. NaOH. The organic phase is dried with $MgSO_4$ and stripped. This gives a brown solid which is crystallized from EtoAc/hexane.
Yield = 0.75g = 40.7%
m.p. = 145-147 deg.C dec.

## EXAMPLE 11

4,5-Dichloro-2-(3,4-dichlorophenyl)-1-(ethoxymethyl)pyrrole-3-carbonitrile

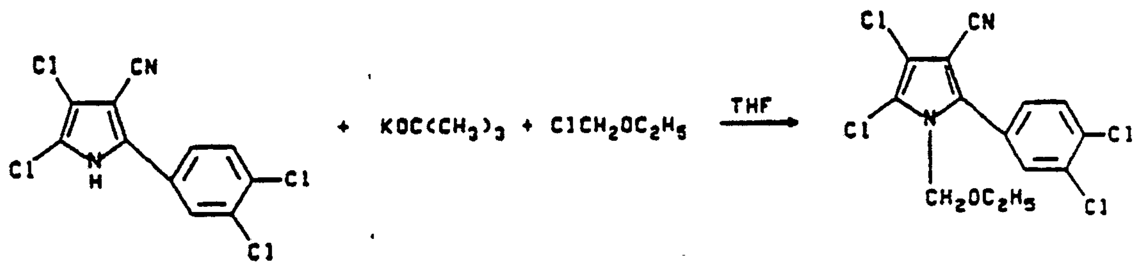


A sample of 4,5-dichloro-2-(3,4-dichlorophenyl)pyrrole-3-carbonitrile (1.0g 0.003 mole) is dissolved in 10 mL of dry tetrahydrofuran. To this solution is added potassium t-butoxide (0.37g, 0.0033 mole) followed by chloromethyl ethyl ether (0.312g, 0.0033 mole). The mixture is stirred for about 1 hour at room temperature and then poured into a large volume of water precipitating the product. The white solid is collected and dried to give 1.0g (91%) with m.p. 128-130°.

## EXAMPLE 12

4-Chloro-3-cyano-2-(p-chlorophenyl)pyrrole

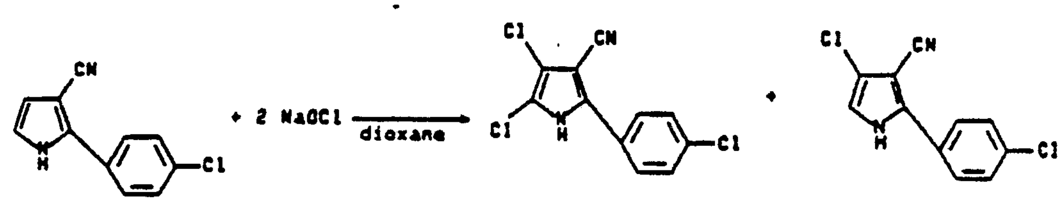


To a magnetically stirred 20°C solution of 17.87g (88.2 mmol, 1.00eq) of 2-(p-chlorophenyl)-3-cyanopyrrole in 800 mL of dioxane is added dropwide 250.15g (13.13g real, 176.4 mmol, 2.00eq) of 5.25 weight % bleach over a period of 30 minutes. After stirring at room temperature for a further 30 minutes, the reaction solution is poured into 2200 mL of water. The resulting mixture is vacuum filtered to remove a small amount of a black solid. The filtrate is acidified to pH 2 with concentrated HCl to produce a brown solid. This solid is vacuum filtered and the collected solids washed with water to give 22.41g of a brown solid. This solid is treated with 100 mL of 5% aqueous sodium hydroxide to dissolve the bulk of the material while leaving a small amount of undissolved black solid. This black solid, dissolved into 100 mL of ethyl acetate, is washed with 75 mL each of 5% aqueous NaOH, water, and sat. aqueous NaCl. The ethyl acetate layer is dired ($MgSO_4$), treated with charcoal, filtered, and then rotary evaporated in vacuo to give 1.10g (5.3% yield) of an orangish brown solid. This solid is recrystallized from an ethyl acetate chloroform mixture to give 0.51g (2.4% yield) of an off-white solid of 4-chloro-3-cyano-2-(p-chlorophenyl)pyrrole. mp 251-253.5°C.

## EXAMPLE 13

Preparation of 5-bromo-2-(3,4-dichlorophenyl)pyrrole-3-carbonitrile

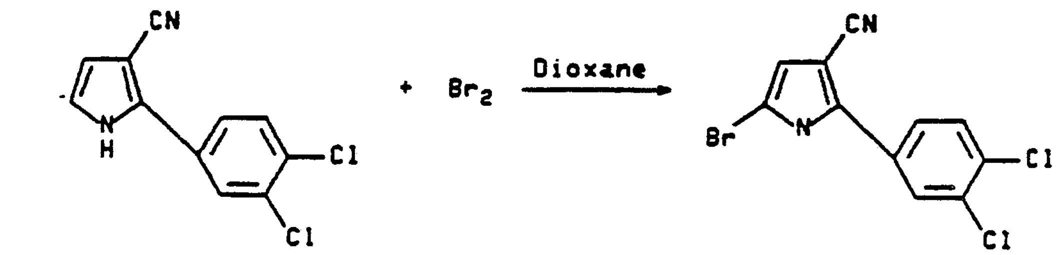


A sample of 2-(3,4-dichlorophenyl)pyrrole-3-carbonitrile (2.0g., 0.008 mole) is dissolved in 100 mL of dioxane by warming to 40-50°. Then the solution is cooled to 30°C and bromine (1.3g, .008 mole) is added. After stirring 1 hour at room temperature the solution is poured into water and a gray solid (2.2g, 88%) is collected. The mp is 233-236°C, decomposition.

In a similiar fashion one can prepare 5-bromo-2-(3,4-dichloro)-3-nitropyrrole starting with 2-(3,4-dichlorophenyl)-3-nitropyrrole.

EXAMPLE 14

Preparation of 5-bromo-4-chloro-2-(3,4-dichlorophenyl)pyrrole-3-carbonitrile

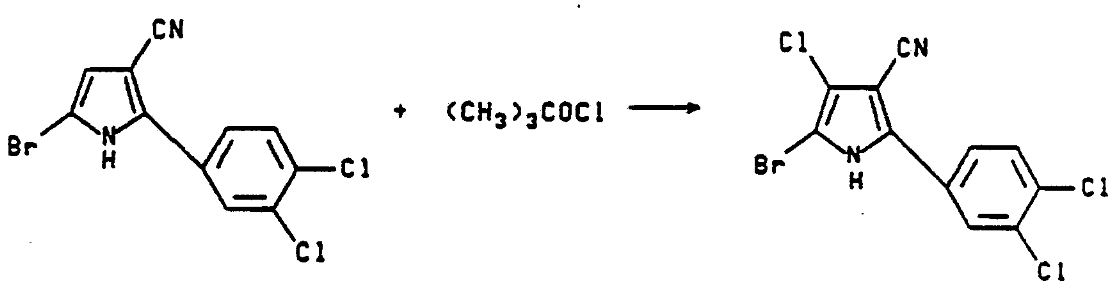

A sample of 5-bromo-2-(3,4-dichlorophenyl)pyrrole-3-carbonitrile (0.158g, 0.005 mole) is dissolved in tetrahydrofuran (5 mL). An equivalent amount of t-butyl hypochlorite is added and the solution stirred overnight. The solution is poured into water and the precipitate (0.052g, 30%) is collected. The mp is >275°C.

In a similiar fashion one can prepare 2-bromo-3-chloro-5-(3,4-dichlorophenyl)-4-nitropyrrole by starting with 2-bromo-5-(3,4-dichlorophenyl)-4-nitropyrrole.

EXAMPLE 15

Preparation of 5-bromo-4-chloro-2-(p-chlorophenyl)pyrrole-3-carbonitrile

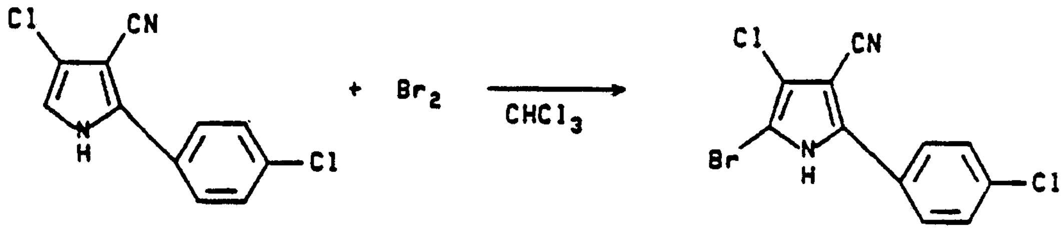


To a magnetically stirred 22°C solution of 0.17g (0.67 mmol., 1.00 equivalent) of 4-chloro-2-(p-chlorophenyl)pyrrole-3-carbonitrile in 100 mL of chloroform is added dropwise over a period of 30 minutes, a solution of 0.20 mL (0.62g, 3.88 mmol., 5.79 equivalent) of bromine in 5 mL of chloroform. The addition produces no exotherm. After stirring at room temperature for 3 1/4 hours, the clear red reaction solution is evaporated in vacuo to give 0.28g of an off-white solid. This solid is slurried with a hexane-methylene chloride mixture to give on vacuum filtration 0.23g of an off-white fluffy solid. mp 262-263°C; dec.

EXAMPLE 16

Preparation of 5-chloro-4-bromo-2-(p-chlorophenyl)pyrrole-3-carbonitrile

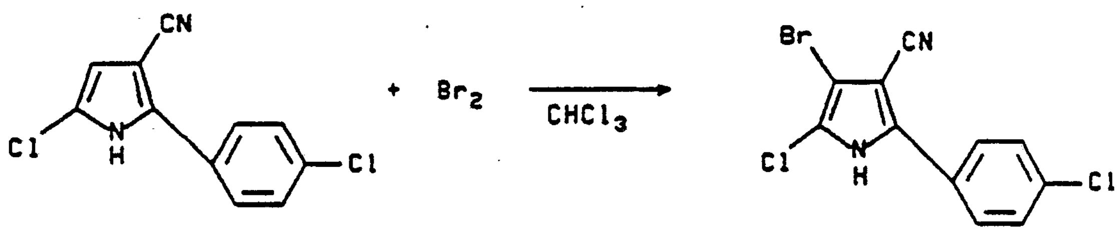


To a magnetically stirred 45°C solution of 1.00g (4.22 mmol., 1.00 equivalent) of 5-chloro-2-(p-chlorophenyl)pyrrole-3-carbonitrile in 300 mL of chloroform is added dropwise over a period of 30 minutes, a solution of 0.40 mL (1.24g, 7.76 mmol., 1.84 equivalent) of bromine in 25 mL of chloroform. The addition produces no exotherm and towards the end of the addition, a small amount of a solid starts to precipitate. After stirring at room temperature for 19 1/2 hours the reaction mixture is evaporated in vacuo to give 1.49g of an orangish white solid. This solid is slurried with a hexane-methylene chloride mixture to give on vacuum filtration 1.33g (100% yield) of a fluffy white solid. mp 250-258°C, dec.

EXAMPLE 17

Preparation of 5-chloro-2-(p-chlorophenyl)pyrrole-3-carbonitrile

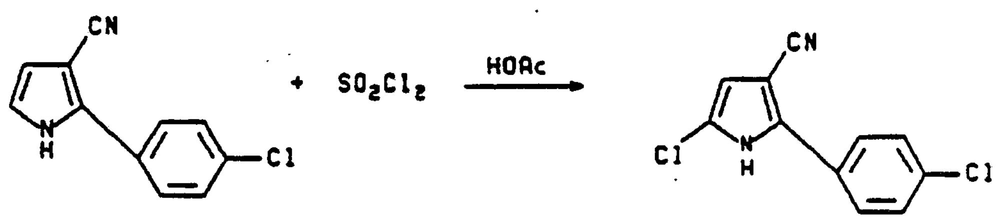


To a 35°C magnetically stirred solution of 2.40g (11.8 mmol., 1.00 equivalent) of 2-(p-chlorophenyl)-pyrrole-3-carbonitrile, and 65 mL of glacial acetic acid is added dropwise by syringe 0.75 mL (1.26g, 9.34 mmol., 0.79 equivalent) of sulfuryl chloride over a period of 5 minutes. Approximately 5 minutes after the completion of the addition, a solid precipitated out of the reaction solution. After stirring at room temperature for 45 minutes, the reaction mixture is filtered and the collected solid is washed well with cold acetic acid to give 2.08g (74% crude yield) of an off-white solid. This solid is recrystallized from 75 mL of hot acetic acid to give 1.63g (58% yield) of 97 wt% pure. Product mp 258.5-261°C.

## EXAMPLE 18

### Preparation of 2-(3,4-dichlorophenyl)-1-methylpyrrole-3-carbonitrile

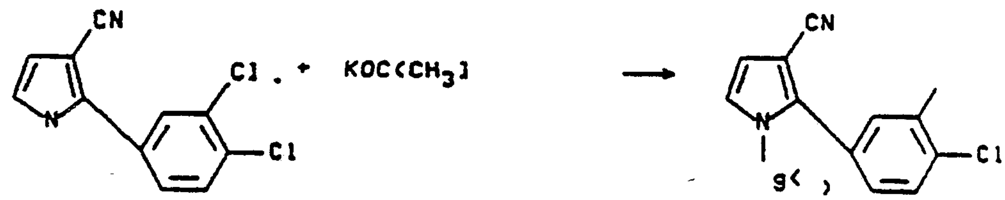

In a 100 mL flask, 2.0 g of 2-(3,4-dichlorophenyl)pyrrole-3-carbonitrile is dissolved in 50 mL of dry THF and 1 equivalent of potassium t-butoxide is added. This gives a slightly cloudy solution. One equivalent of methyl iodide is then added to the mixture by pipette. This leads to a slight lightening of the color. A drying tube is attached to the flask and it is left to stir at ambient temperature overnight.

The next morning there is a slight light-coloured precipitate in the flask. 50 mL of water is then added and the solution becomes clear before a solid precipitates out of the solution. This solid is filtered out of the solution and compared to the starting material by TLC (25% ethyl acetate/hexane). This indicates a new single spot which is faster moving than the starting material. It is dried in a vacuum oven at 50 deg. C overnight. The product yield is 1.31g or 62% yield and has a melting point of 140-142°C.

## EXAMPLE 19

### Preparation of 4,5-dichloro-2-(3,4-dichlorophenyl)-1-methylpyrrole-3-carbonitrile

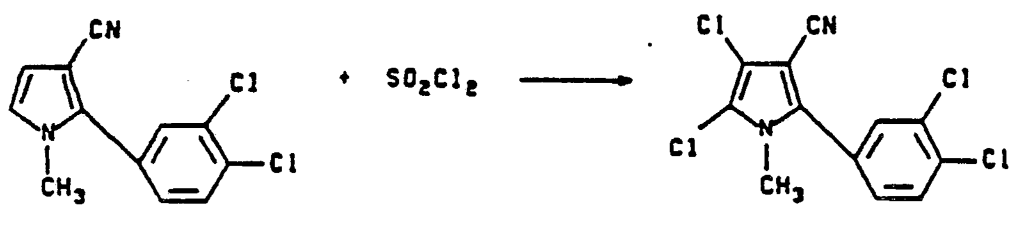

In a 50 mL round bottom flask, 0.5g of 2-(3, 4-dichlorophenyl)-1-methylpyrrole-3-carbonitrile is mixed with 35 mL of glacial acetic acid. The mixture is warmed slightly with a heat gun to dissolve all of the pyrrole.

To this clear solution is added 2 eq. of sulfuryl chloride by pipette. The solution is left to stir at room temperature for 12 hours.

After 12 hours the solution is poured into 50 mL of water, resulting in a white precipitate. This is filtered out and dried in a vacuum over at 50°C for 3 hours.

The resulting solid is identical by TLC, (25% ethyl acetate/ hexane), and infrared analysis to the product of Example 9. Product yield is 0.36 (56%).

EXAMPLE 20

Preparation of 4,5-Dichloro-2-(3,4-dichlorophenyl)-1-(2-hydroxyethyl)-pyrrole-2-carbonitrile

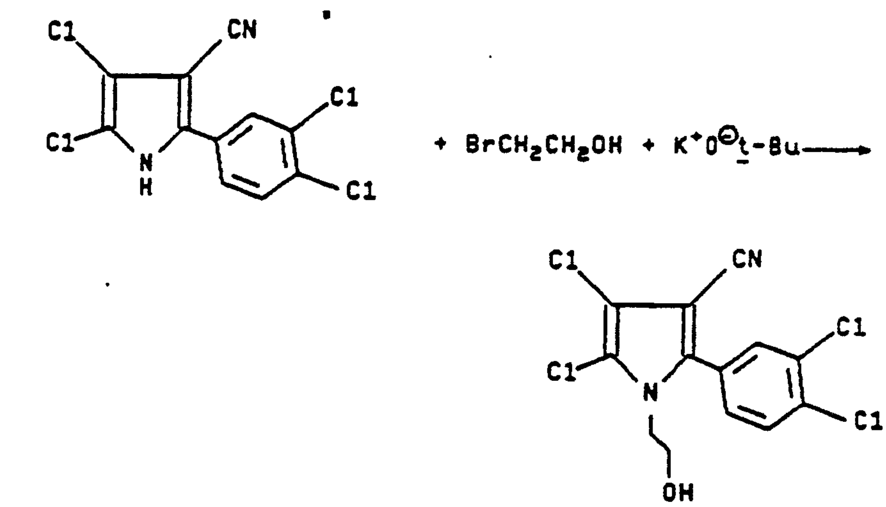


To a stirred mixture of 2.0 g (6.5 mmol) of 4,5-dichloro-2-(3,4-dichlorophenyl)-pyrrole-3-carbonitrile and 0.88 g (7.8 mmol) of potassium tert-butoxide heated at reflux in 50 mL of dioxane is added 0.98 g (7.8 mmol) of bromoethanol. The mixture is stirred at reflux for 12 hours, cooled, diluted with 50 mL of water, and extracted several times with chloroform. The combined chloroform extracts are dried over magnesium sulfate and concentrated in vacuo to leave a solid which, on warming and dissolving in ethyl acetate, deposits on cooling mostly starting pyrrole. Concentration of the mother liquor and recrystallization of the residual solid from 20% ethyl acetate in hexane gives 0.31 g of a white solid, mp 143-145°C; IR 5077A.

| | | | | |
|---|---|---|---|---|
| Anal. Calc'd for $C_{16}H_{23}NO_4$; | C, 44.57, | H, 2.29; | N, 8.00; | Cl, 40.57. |
| Found: (Agm 33139): | C, 44.77; | H, 2.29; | N, 8.06; | Cl, 40.14. |

EXAMPLE 21

Preparation of 4,5-dichloro-2-(3,4-dichlorophenyl)pyrrole-1,3-dicarbonitrile

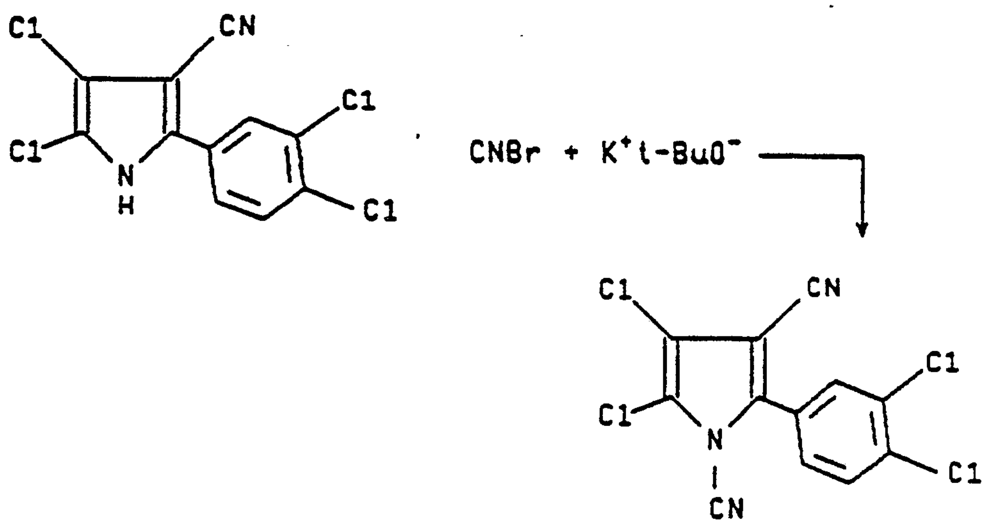


Potassium t-butoxide (617 mg, 55 mmol) is added in portions to a solution of 3-cyano-4,5-dichloro-2-(3,4-dichlorophenyl)pyrrole (1.52 g, 5 mmol) in anhydrous THF (20 mL). After 30 minutes, a solution of cyanogen bromide (583 mg, 5.5 mmol) in THF (1 mL) is added. The reaction mixture is stored at room temperature overnight. The solvent is removed in a rotary evaporator. The residue is treated with water and extracted with ethyl acetate. The organic layer is washed with water and saturated sodium chloride and dried ($MgSO_4$). Evaporation and crystallization of the residue from ethyl acetate gives while crystals (1.07 g); mp 250.5-252.0°C; IR (nujol) 2255, 2245 $cm^{-1}$ (CN); $^{13}C$ NMR (DMSO-$d_6$) 102.7 (N-CN), 113.7 (3-CN); Mass spectrum 331.9 (M+1).

| | | | | |
|---|---|---|---|---|
| Anal. Calc'd for $C_{12}H_3CP_4N_3$ (330.99); | C, 43.54; | H, 0.91; | N, 12.70; | Cl 42.85. |
| Found: | C, 4362; | H, 0.93, | N, 12.63; | Cl 41.95. |

## EXAMPLE 22

Preparation of 4,5-Dichloro-2-(3,4-dichlorophenyl)-1-(3-iodo-2-propynyl)-pyrrole-3-carbonitrile

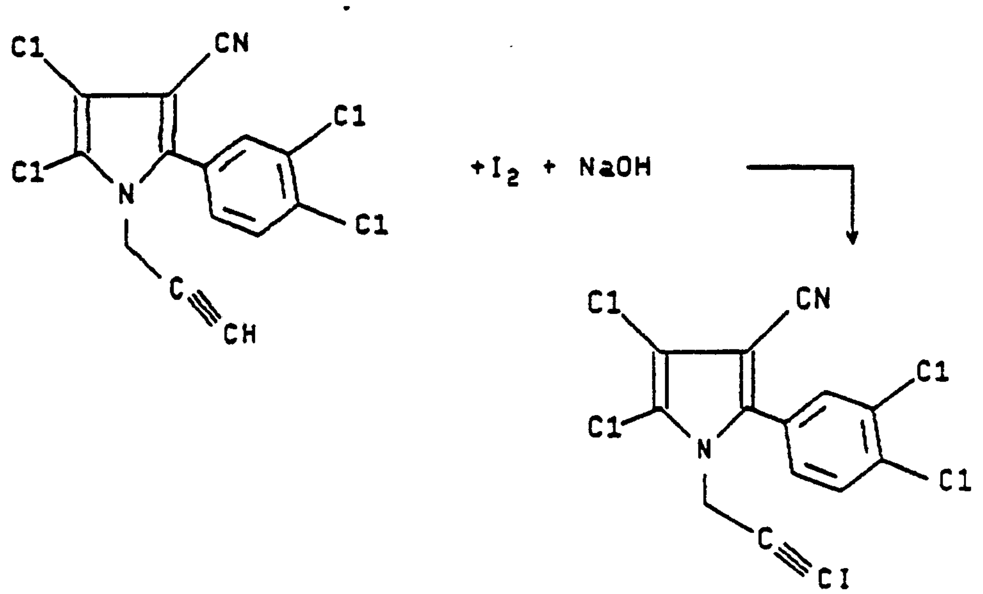


To a stirred mixture of 1.91 g (5.5 mmol) of 4,5-dichloro-2-(3,4-dichlorophenyl)-1-(2-propynyl)-pyrrole-3-carbonitrile in 500 mL of methanol is added 69 mL of 10% aqueous sodium hydroxide and then 0.70 g (2.7 mmol) of iodine. The mixture is stirred for 12 hours and then acidified and diluted with 200 mL of water. The precipitated solids are collected and recrystallized from methanol to afford 0.51 g while crystals, m.p. 115-116°C.

This reaction is also applicable to the conversion of any of the formula III, IV, V, VI or VII substituted N-alkynylarylpyrroles of the present invention to N-substituted 3-iodo-2-propynyl arylpyrroles of said invention.

## EXAMPLE 23

Preparation of 2-(3,4-dichlorophenyl)-4,5-diiodopyrrole-3-carbonitrile

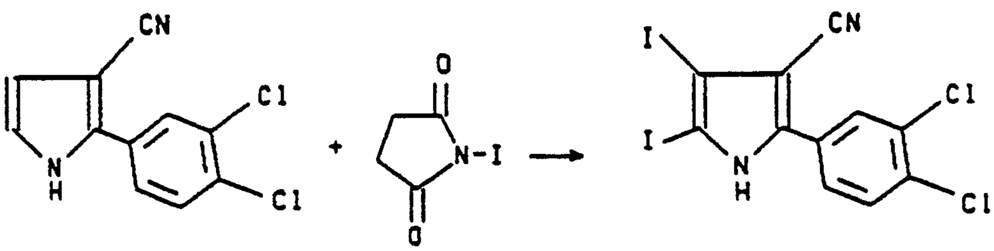

N-iodosuccinimide (5.7 g, .0254 mol,) is added slowly to a solution of 2-(3,4-dichlorophenyl)-pyrrole-3-carbonitrile (3.0 g, .0127 mol) in 100 ml of THF. The reaction is stirred several hours at 25°C until thin layer chromatography (silica gel; 100:100:1-ether:petrolium ether:acetic acid) shows completion. The mixture is evaporated *in vacuo* to give a residue containing the pyrrole and succinimide. The crude solid is dissolved in 500 mL of ether and shaken with 5 x 400 mL of water to remove the succinimide. The ether is dried over $Na_2SO_4$ and evaporated *in vacuo* to leave 2.0 g (32.3%) of a grey-brown solid with mp >230° (loses purple vapors).

## EXAMPLE 24

Preparation of 2-phenyl-1-pyrroline-4-carbonitrile

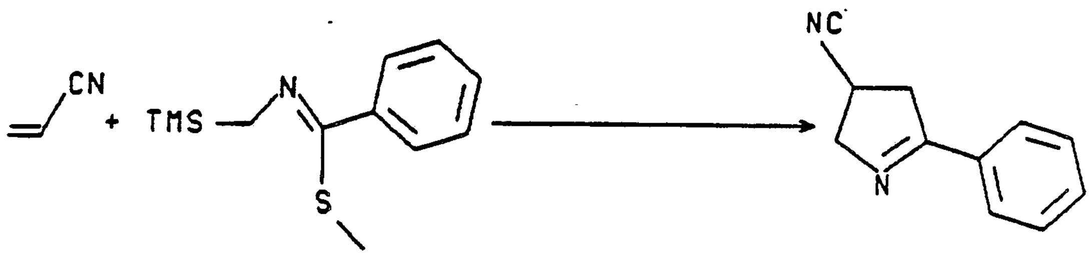

A solution of acrylonitrile (0.65 mL; 0.01 mol) and N-(trimethylsilyl)methyl-S-methyl-benzenethioimidate (2.4 g; 0.01 mol) in THF (100 mL) is cooled to -5°C in an ice-acetone bath. Under a nitrogen purge, a solution of tetrabutylammonium fluoride (1.0 mL of a 1 N solution in THF) and THF (20 mL) is added dropwise over 30 minutes The solution is stirred another 30 minutes at -5°C, and then allowed to warm slowly to ambient. Stirring is continued another 18 hours, and then solvent is removed under reduced pressure. The residue is partitioned between ether/water and the water layer extracted with fresh ether. The combined organic layer is washed with water, then saturated sodium chloride. The solution is dried over $MgSO_4$, and cooling the filtrate causes precipitation of an off-white solid (1.2 g; 70% theoretical yield) whose spectral characteristics are identical to the material described by Tsuge [J. Org. Chem. 52, 2523 (1987)].

| | | | |
|---|---|---|---|
| Calcd. for $C_{11}H_{10}N_2$: | C, 77.65; | H, 5.88; | N, 16.47. |
| Found: | C, 77.55; | H, 5.83; | N, 16.39. mp = 95-97°C. |

## EXAMPLE 25

Preparation of 2-phenyl-pyrrole-4-carbonitrile

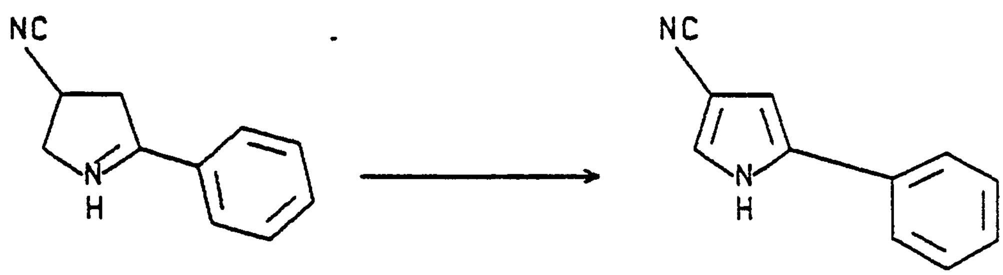

Under a nitrogen purge 2,3-dichloro-5,6-dicyano-1,4-bonzoquinone (0.23 g; 0.001 mol) and 2-phenyl-1-pyrroline-4-carbonitrile (0.17 g; 0.001 mol) is dissolved in 1,2-dimethoxyethane (13 mL) to form a clear orange solution. Pyridine (0.08 mL; 0,001 mol) is added in a single portion, causing a slight exotherm (to ca. 28°C) and an immediate formation of a green/grey precipitate. The suspension is stirred at room temperature for 18 hours during which time much of the solvent evaporates. The brownish semi-solid residue is partitioned between ether and a half-saturated solution of sodium carbonate. The red-brown aqueous layer is extracted twice with ether and the combined ether layer is washed with fresh water, then saturated sodium chloride. After drying with $MgSO_4$, solvent is removed under reduced pressure to obtain a white semi-solid. This material was recrystallized from ethylene dichloride (DARCO treatment) to yield

lavender crystals (0.1 g).

The identical product is obtained directly in a single step by condensing α-chloroacrylonitrile and N-(trimethylsilyl)methyl-S-methyl-benzenethioimidate using tetrabutylammonium fluoride catalysis (analogous to the preparation of 2-phenyl-1-pyrroline-4-carbonitrile described previously).

| | | | |
|---|---|---|---|
| Calcd. for $C_{11}H_8N_2$: | C, 78.57, | H, 4.76; | N, 16.67. |
| Found: | C, 78.65; | H, 4.70; | N, 16.43. m.p. - 155-158°C. |

## EXAMPLE 26

Preparation of 2,4-dibromo-5-phenyl pyrrole-3-carbonitrile

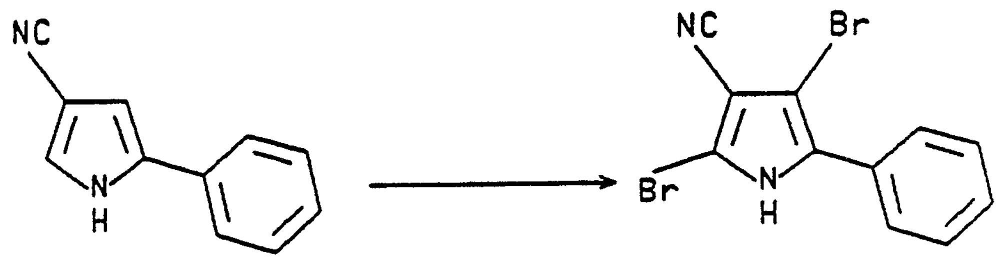

Under a nitrogen purge, a solution of bromine (0.6 mL; 0.012 mol) in $CHCl_3$ (5 mL) is added dropwise over 20 minutes to a stirring solution of 2-phenyl-pyrrole-4-carbonitrile (0.84 g; 0.05 mol) in $CHCl_3$ (20 mL). The resulting solution is stirred 18 hours at room temperature, then solvent is removed under reduced pressure to obtain a solid which is recrystallized from $C_2H_4Cl_2$ (DARCO treatment), yielding the desired final product (0.6 g), m.p. = 239-242°C.

| | | | | |
|---|---|---|---|---|
| Calcd. for $C_{11}H_6Br_2N_2$: | C, 40.49; | H, 1.84; | Br, 49.08; | N, 8.59. |
| Found: | C, 39.88; | H, 1.87; | Br, 48.81; | N, 8.48. |

By the procedure described in Example 24, 25 and 26, 2,4-dibromo-5-(p-chlorophenyl)pyrrole-3-carbonitrile, m.p. 270-272°C (dec.) is also prepared.

## EXAMPLE 27

3′,4′-Dichloro-3-(1,3-dioxolan-2-yl)-propiophenone

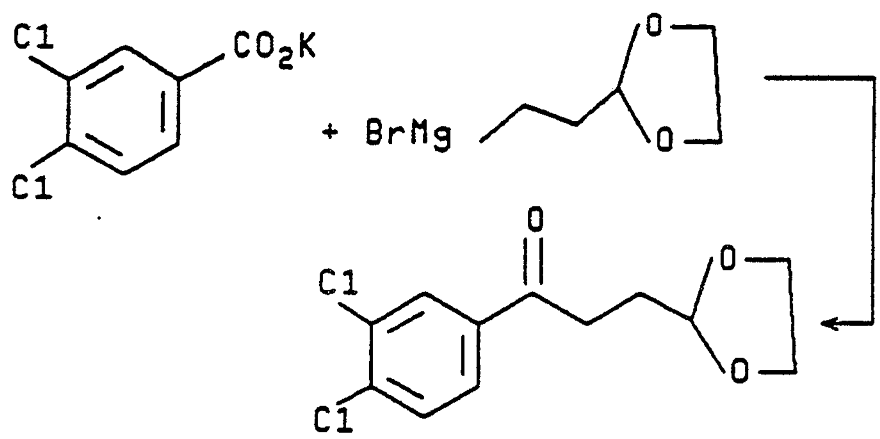

To a rapidly stirring mixture of magnesium turnings (0.64 g, 26 mmol) in 10 mL of tetrahydrofuran at 25°C in a 100 mL three-neck round bottom flask equipped with a thermometer, a 60 mL addition funnel, and a nitrogen inlet is added dropwise 2-(2-bromoethyl)-1,3-dioxolane (4.7 g, 26 mmol) in 40 mL of tetrahydrofuran. The rate of addition is adjusted so as to maintain the reaction temperature below 50°C. The reaction is then allowed to stir for 1 hour at 25°C. 120 mL of tetrahydrofuran is mixed with potassium 3,4-dichlorobenzoate (5.0 g, 22 mmol) under a blanket of nitrogen. The Grignard solution is then quickly decanted away from the unreacted magnesium turnings, and added dropwise to the rapidly stirring potassium benzoate suspension. The reaction is then allowed to stir for 24 hours at 25°C. Fifty mL of diethyl ether and 15 mL of 3N hydrochloric acid are added to the reaction mixture and the layers separated. The organic layer is washed with saturated aqueous sodium bicarbonate until neutral followed by one washing with 10 mL of brine. Drying over sodium sulfate, and rotary evaporation yields a beige semisolid which is chromatographed over silica gel using 3:1 hexane-ethyl acetate as eluent to give the keto-acetal (4.3 g, 60%) as a white solid, m.p. 115-117°C.

## EXAMPLE 28

Preparation of 3-(3,4-dichlorobenzoyl)propionaldehyde

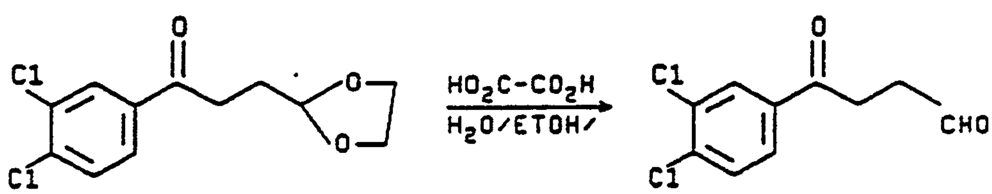


Ten grams (26 mmol) of 3′,4′-dichloro-3-(1,3-dioxolan-2-yl)-propiophenone is added to 30 mL of 0.2M oxalic acid (made by dissolving 0.9 g of oxalic acid dihydrate in 30 mL of water) and 5 mL of ethanol. The mixture is refluxed for 1 hour and then allowed to cool. Most of the ethanol is rotary evaporated off and 100 mL of diethyl ether is added along with 20 mL of saturated aqueous sodium bicarbonate. The layers are separated and the organic phase is dried over magnesium sulfate. Rotary evaporation yields a viscous yellow oil which is chromatographed over silica gel using 3:1 hexane-ethyl acetate to give the keto-aldehyde (6.3 g, 75%) as a white solid.

## EXAMPLE 29

Preparation of 2-(3,4-dichlorophenyl)pyrrole

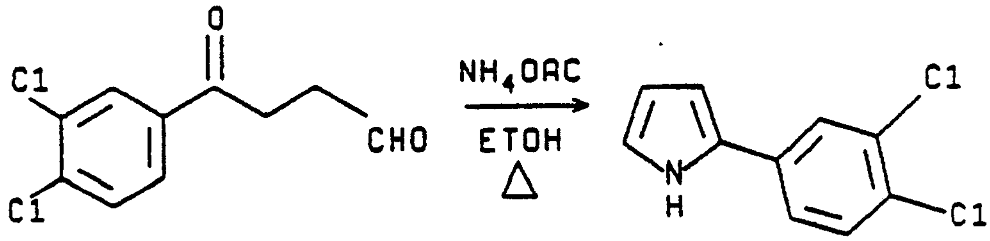


To a suspension of 3-(3,4-dichlorobenzoyl) propionaldehyde (6 g, 26 mmol) in 60 mL of absolute ethanol is added ammonium acetate (4 g, 52 mmol). The reaction is refluxed for 20 minutes and allowed to cool. Most of the ethanol is rotary evaporated and 200 mL of 1:1 dichloromethane-diethyl ether along with 50 mL of water is added. The layers are separated and the organic phase is dried over sodium sulfate. Rotary evaporation yields a dark brown oil which is chromatographed over silica gel using 3:1 hexane-ethyl acetate as eluent to give the pyrrole (4.6 g, 83%) as a light brown solid, m.p. 49-51°C.

EXAMPLE 30

Preparation of 5-(3,4-dichlorophenyl)pyrrole-2-carboxaldehyde

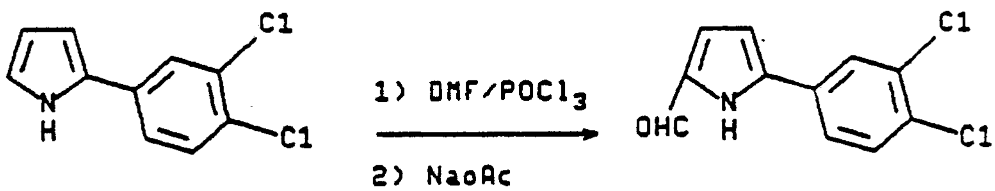


To 10 mL of dimethylformamide stirring under nitrogen in a 50 mL round bottom flask is added phosphorus oxychloride (0.6 mL, 6.5 mmol) dropwise via syringe. The solution, warms and becomes light yellow in color. It is allowed to stir for 20 minutes before the portionwise addition of 2-(3,4-dichlorophenyl)-pyrrole (1 g, 4.7 mmol). The beige suspension which results is allowed to stir for 30 minutes before being heated to 50°C for 40 minutes. A solution of sodium acetate (10 g, 122 mmol) in 15 mL of water is added to the cooled reaction which is then allowed to stir for 20 minutes. A beige precipitate is filtered off from the reaction mixture and air-dried for 20 hours to give the essentially pure aldehyde (1.1 g, 95%), mp > 200°C.

EXAMPLE 31

Preparation of 5-(3,4-dichlorophenyl)pyrrole-2-carbonitrile

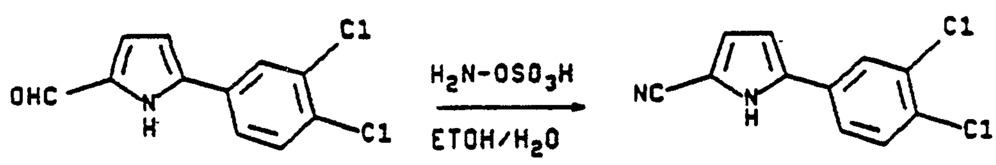

To a suspension of 5-(3,4-dichlorophenyl)pyrrole-2-carboxaldehyde (1.5 g, 6.2 mmol) in 20 mL of water and 20 mL of ethanol, is added hydroxylamine-O-sulfonic acid (0.7 g, 6.2 mmol). The reaction is refluxed for 1 hour during which time a gray precipitate appears. After being allowed to cool, the reaction is filtered to give essentially pure nitrile (1.5 g, 99%) as a gray solid, m.p. 170-171°C.

## EXAMPLE 32

Preparation of 3,4-dibromo-5-(3,4-dichlorophenyl)pyrrole-2-carbonitrile

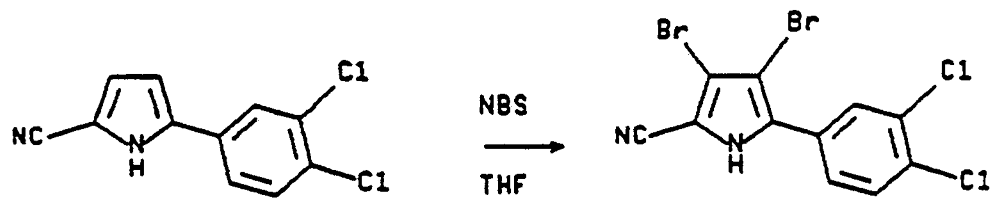


To a solution of 5-(3,4-dichlorophenyl)pyrrole-2-carbonitrile (0.5 g, 2.1 mmol) in 20 mL of tetrahydrofuran under nitrogen is added portionwise N-bromo-succinimide (0.8 g, 4.2 mmol). The reaction is stirred at 25°C for 30 minutes before the addition of 10 mL of water and 40 mL of diethyl ether. The layers are separated and the organic layer dried over sodium sulfate. Rotary evaporation is followed by chromatography over silica gel using 3:1 hexane-ethyl acetate as eluent to afford the dibromopyrrole (0.5 g, 60%) as a brown solid, m.p. > 250°C.

## EXAMPLE 33

Preparation of 4-phenylpyrrole-3-carbonitrile

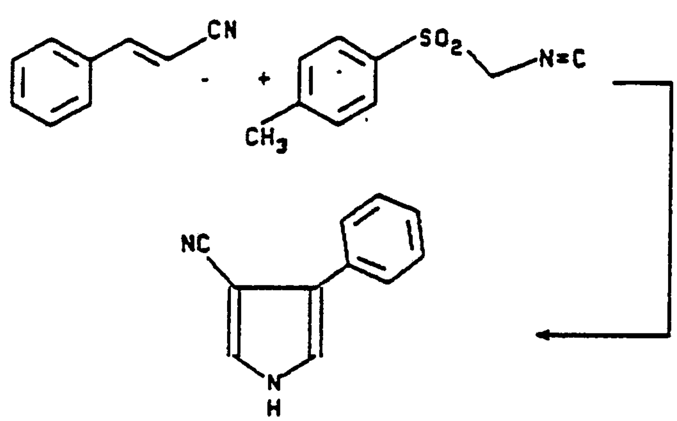

To a mixture of 5.0 g (39 mmol) of cinnamonitrile and 7.6 g (39 mmol) of (p-tolylsulfonyl)methyl isocyanide in 35 mL of DMSO and 65 mL of ether is added over a 20 minute period a suspension of 1.86 g of a 60% oil suspension of sodium hydride (1.11 g; 46 mmol) in 80 mL of ether. The reaction mixture is maintained under nitrogen for an hour and then diluted with ether and water. The ether layer is separated, dried over magnesium sulfate, and concentrated *in vacuo*. The resulting oil is chromatographed on silica gel using 1:1 chloroform ethyl acetate to give 2.5 g of cream-colored solids. Recrystallization from ether-hexane affords 1.15 g, m.p. 123-125°C; NMR M86-1077.

Lit.: Tet. Letters 5337 (1972): m.p. 128-129°C.

EXAMPLE 34

Preparation of 2,5-dichloro-4-phenylpyrrole-3-carbonitrile

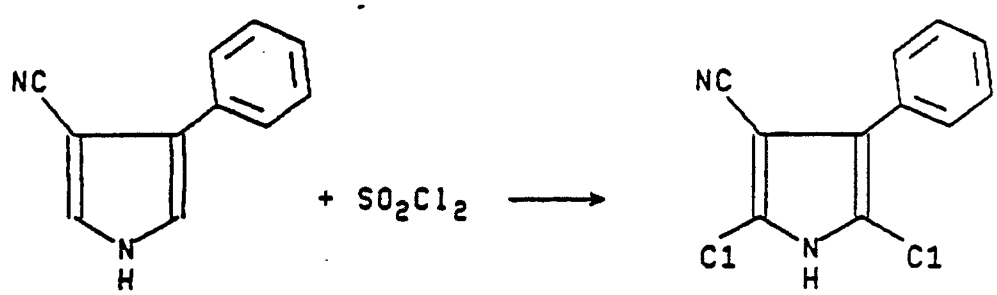

To a stirred mixture of 0.66 g (3.9 mmol) of 4-phenylpyrrole-3-carbonitrile in 20 mL of dry THF cooled to 6°C with an ice-water bath is added from a syringe 0.66 mL (1.11 g; 8.2 mmol) of sulfuryl chloride over a 4 minute period. The mixture is maintained at 5-10°C for an additional 45 minutes and then stirred an additional 30 minutes with the ice bath removed. After the reaction mixture is poured into 80 mL of ethyl acetate and 40 mL of water, the organic phase is separated, washed with water, and dried over sodium sulfate. Filtration through a short column of silica gel, rinsing with ethyl acetate, and concentration of the combined filtrated in vacuo gives 0.95 g of dark solid. Recrystallization from chloroform gives 0.42 g of off-white crystals, m.p. 195-196°C (dec.).

| | | | | |
|---|---|---|---|---|
| Anal. Calcd for $C_{11}H_6Cl_2N_2$: | C, 55.72; | H, 2.55; | N, 11.82; | Cl, 29.91. |
| Found: | C, 55.66; | H, 2.65; | N, 11.69; | Cl, 29.97. |

Following the procedures of Examples 33 and 34, the following analogs are prepared. For the synthesis of 2,6-dibromo-4-(p-chlorophenyl)pyrrole-3-carbonitrile, the procedure of Example 33 is followed using bromine in dioxane to replace sulfuryl chloride and tetrahydrofuron.

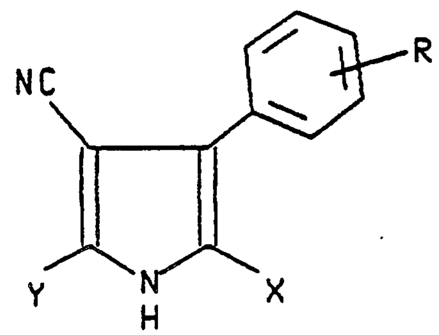

| R | X | Y | m.p. °C |
|---|---|---|---|
| 4-Cl | Cl | Cl | 237-240 (dec.) |
| 4-$CH_3$ | Cl | Cl | 103-206 |
| 4-Cl | Br | Br | > 245° |

EXAMPLE 35

Ethyl 4-(p-chlorophenyl)-pyrrole-3-carboxylate

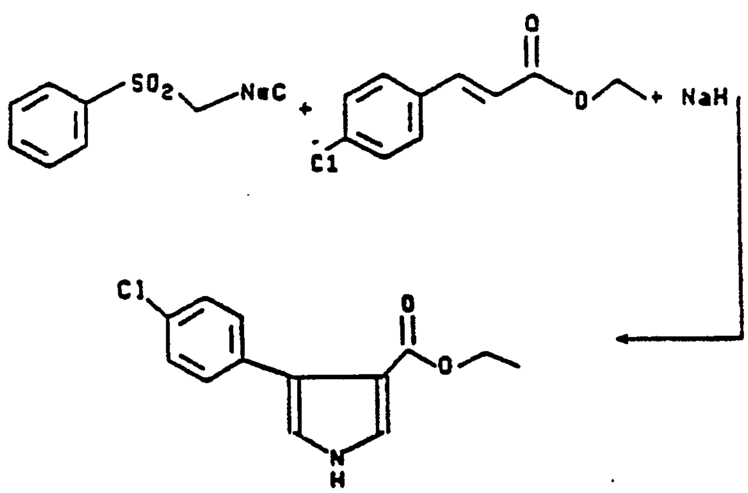

To a mixture of 5.63 g of a 60% sodium hydride/oil suspension in 200 mL of dry ether under nitrogen is added from an additional funnel a mixture of 23.5 g (122 mmol) of ethyl p-chlorocinnamate and 19.4 g (122 mmol) of (p-tolylsulfonyl)methyl isocyanide in solution in 180 mL of ether and 80 mL of dimethylsulfonide. The addition time is about 20 minutes and results in gentle refluxing of the mixture. After another 10 minutes stirring, the mixture is diluted with 100 mL of water. The mixture is extracted four times with ether which is then dried over magnesium sulfate followed by concentrated in vacuo. The resulting solid is recrystallized from ethylene dichlorite to give 7.8 g of crystals, m.p. 137-138°C.

| | | | | |
|---|---|---|---|---|
| Anal. Calcd for $C_{13}H_{12}ClNO_2$: | C, 62.53; | H, 4.81; | N, 5.61; | Cl, 14.23. |
| Found: | C, 61.31, | H, 5.12; | N, 5.32; | Cl, 14.57. |

Concentration of the mother liquor for the crystallization leaves additional crude ester which is carried on to the saponification step.

## EXAMPLE 36

Preparation of 3-(p-chlorophenyl)-pyrrole

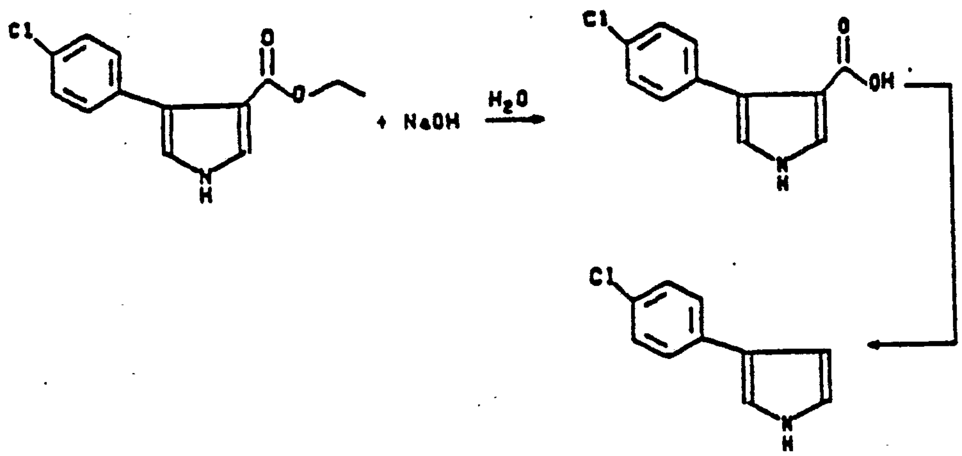


A mixture of 22.0 g of crude ethyl 4-(p-chlorophenyl)-pyrrole-3-carboxylate from the recrystallization mother liquor and the recrystallized product from the previous step is stirred at reflux with 150 mL of 10% aqueous sodium hydroxide for 2.5 hours. The mixture is cooled, extracted with ether, and acidified to give a precipitate which on collection and drying weighs 11.6 g.

A mixture of 10.5 g of the acid in 100 mL of $\beta$-ethanolamine is heated at reflux for three hours. After cooling, the mixture is poured over 400 mL of ice and the resulting mixture is extracted four times with chloroform. The chloroform solution, after drying over magnesium sulfate and treatment with activated charcoal, is concentrated in vacuo to leave a brown solid. Chromatography on silica gel using 1:1 ethyl acetate hexane gives 4.0 g of a white solid, m.p. 117-118°C.

## EXAMPLE 37

Preparation of 3-(p-chlorophenyl)-pyrrole-2-caboxaldehyde

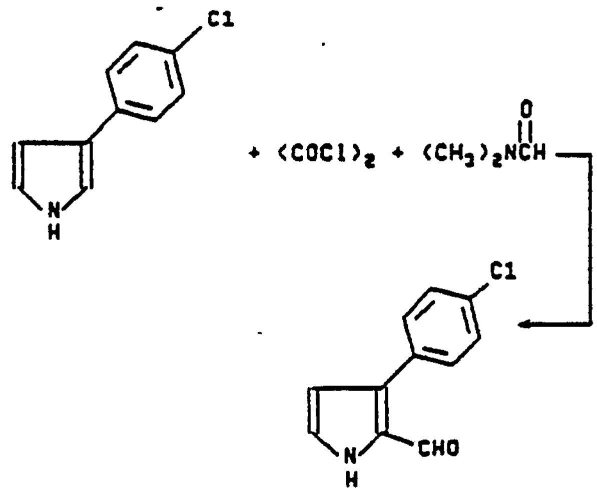

To a mixture of 0.86 g (12 mmol)•of dimethylformamide in 10 mL of ethylene dichloride maintained under nitrogen and cooled in an ice bath is added 1.49 g (12 mmol) of oxalyl chloride in 10 mL of ethylene dichloride over a period of 25 minutes. The ice bath is removed, the mixture is stirred an additional 15 minutes and recooled in an ice bath. To this mixture is added 1.5 g (8.5 mmol) of 3-(p-chlorophenyl)-pyrrole in 25 mL of ethylene dichloride over a 20 minute period. The ice bath is removed and after an additional 30 minutes of stirring, the mixture is poured into 50 mL of ice-water and 6 mL of 50% sodium hydroxide. The resulting mixture is extracted with ether and with chloroform and the combined organic mixture is dried over magnesium sulfate and concentrated in vacuo. Purification of the resulting solid by chromatography on silica gel using 1:1 ethyl acetate hexane gives 0.63 g of off-white solid which is used directly for conversion to 3-(p-chlorophenyl)-pyrrole-2-carbonitrile.

## EXAMPLE 38

Preparation of 3-(p-chlorophenyl)-pyrrole-2-carbonitrile

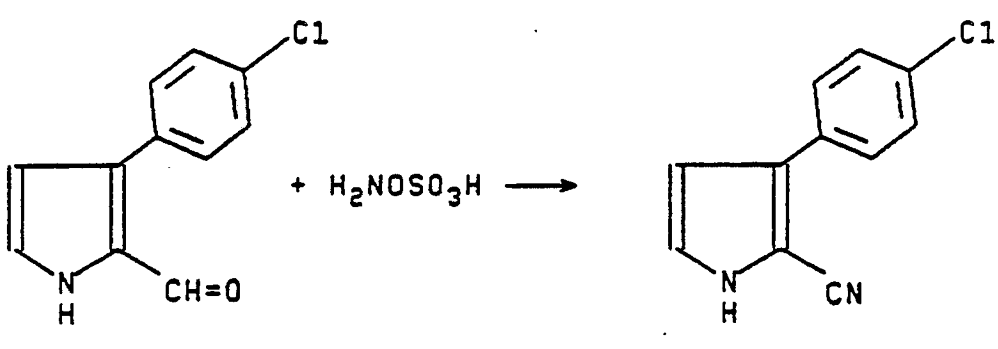

A mixture of 0.63 g (3.1 mmol) of 3-(p-chlorophenyl)-pyrrole-2-carboxaldehyde in 10 mL of water is stirred and ice-cooled while 0.52 g (4.6 mmol) of hydroxylamine-O-sulfonic acid in 10 mL of water is slowly added. After the addition, the cooling bath is removed and the mixture is heated for 25 minutes. On cooling, the resulting solid is collected and shown, by NMR, to be a mixture of product and starting aldehyde. This mixture is reacted in the same manner with an additional 0.49 g (4.2 mmol) of hydroxylamine-O-sulfonic acid in a total of 30 mL of water. The mixture is heated at 60-70°C for 2 hours. The mixture is cooled and the resulting solids are collected and purified by chromatography or silica gel using 1:1 ethyl acetate hexane to give 0.40 g of pink solid, m.p. 114-115°C.

## EXAMPLE 39

Preparation of 4,5-Dibromo-3-(p-chlorophenyl)-pyrrole-2-carbonitrile

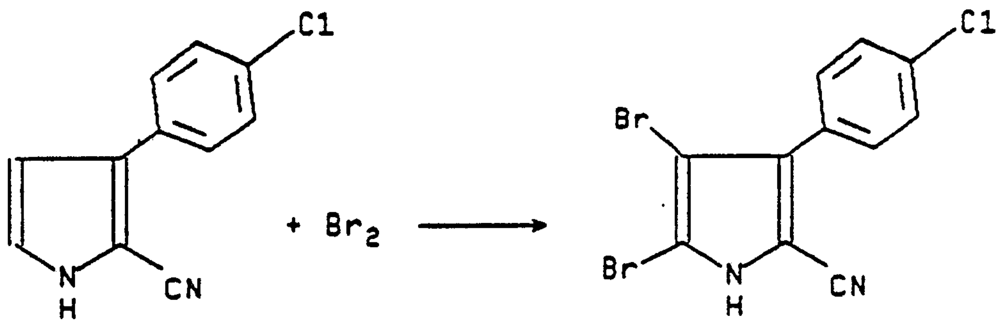

To a mixture 0.40 g (2.0 mmol) of 3-(p-chlorophenylpyrrole)-2-carbonitrile in 25 mL of chloroform is added 0.63 g (4.0 mmol) of bromine. After 20 minutes, the precipitate which forms is collected and recrystallized from ethyl acetate to give 0.21 g of pink crystals, m.p. > 250°C.

| | | | | | |
|---|---|---|---|---|---|
| Anal. Calcd for $C_{11}H_5Br_2ClN$: | C, 36.62; | H, 1.39; | Br, 44.38; | Cl, 9.85; | N, 7.77. |
| Found: | C, 36.92; | H, 1.32; | Br, 44.62; | Cl, 9.88; | N, 7.50 |

## EXAMPLE 40

Preparation of Ethyl 5-bromo-4-(p-chlorophenyl)pyrrole-3-carboxylate

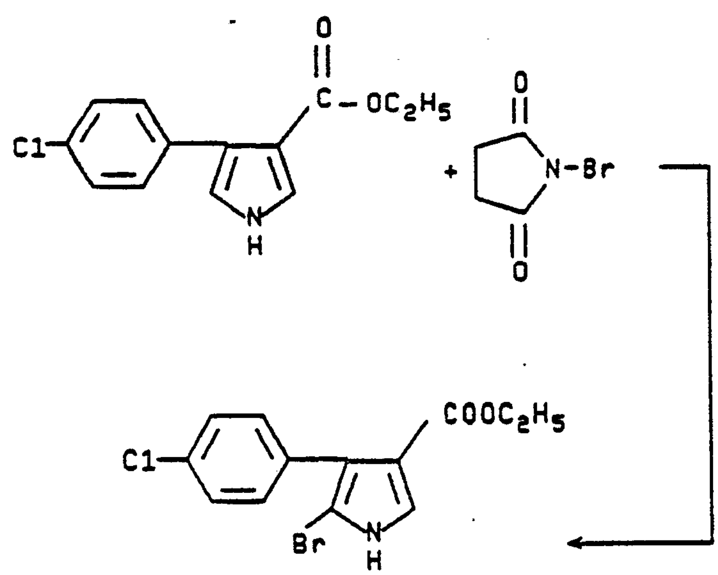

Ethyl 4-(p-chlorophenyl)pyrrole-3-carboxylate (1.6 g., 0.0064 mmol) is dissolved in tetrahydrofuran (40

mL). N-bromosuccinimide (1.14 g., 0.0064 mmol) is added in small portions at 25-28°C. After the addition is complete, the solution is stirred overnight at room temperature. The solution is concentrated in vacuo and the solid residue partioned between water and ether. The ether layer is separated and dried over magnesium sulfate. Work-up of the ether extract leaves 1.9 g (90%) of a white solid which is purified by stirring with a mixture of 80/20 hexane/ethyl acetate. The insoluble solid (1.3 g, 62%) is collected and has m.p. 161-164°C.

| | | | | | |
|---|---|---|---|---|---|
| Calcd for $C_{13}H_{11}BrClNO_2$: | C, 47.50; | H, 3.34; | N, 4.26; | Br, 24.33; | Cl, 10.80. |
| Found: | C, 47.39; | H, 3.38; | N, 4.12; | Br, 24.29; | Cl, 10.77 |

## EXAMPLE 41

Preparation of 5-bromo-4-(p-chlorophenyl)pyrrole-3-carboxylic acid

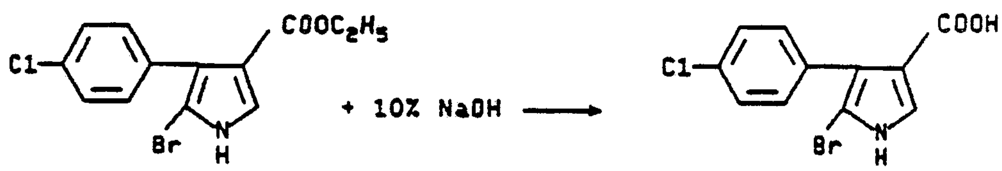


Ethyl 5-bromo-4-(p-chlorophenyl)pyrrole-3-carboxylate (15 g., 0.045 mmol) is added to 200 mL of 10% sodium hydroxide and the slurry heated to reflux. After everything appears to dissolve the mixture is refluxed an additional 40 minutes. The mixture is cooled, filtered and the filtrate acidified. The white precipitate (8.0 g, 58%) is collected and dried. The solid has m.p. >205°C and an NMR ($d_6$-DMSO) which showed a pyrrole proton at 7.52 (d). The mass spectrum is also consistent for a monobrominated compound.

## EXAMPLE 42

Preparation of 2-bromo-3-(p-chlorophenyl)pyrrole

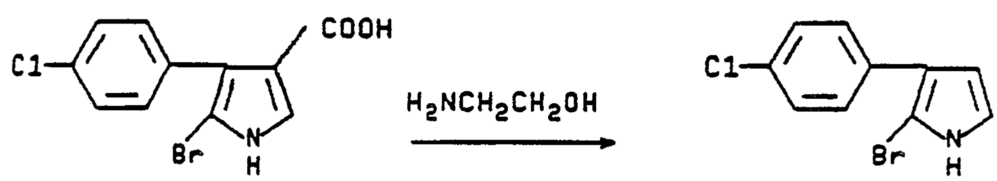


5-bromo-4-(p-chlorophenyl)pyrrole-3-carboxylic acid (8.0 g., 0.026 mmol) is added to aminoethanol (24 mL) and the slurry slowly warmed to 110-120°C and held at that temperature for 1 hour. The solution is cooled and poured into water and extracted with ether. The ether extract, by thin layer chromatography (75/25, hexane/ethyl acetate), shows a major fast moving spot and a slower moving minor component. Work-up of the ether leaves a dark solid (4.0 g., 56%) which is 2-bromo-3-(p-chlorophenyl)pyrrole and is used immediately to prepare 5-bromo-4-(p-chlorophenyl)pyrrole-2-carbonitrile.

## EXAMPLE 43

Preparation of 5-bromo-4-(p-chlorophenyl)pyrrole-2-carbonitrile

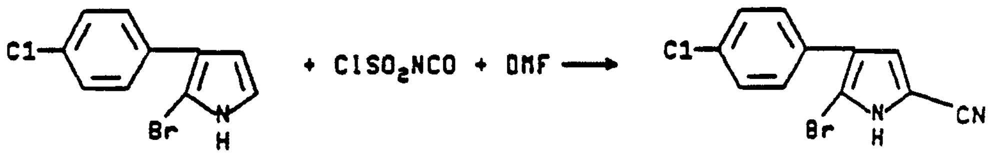


A freshly prepared sample of 2-bromo-3-(p-chlorophenyl)pyrrole (4.0 g., 0.015 mmol) is dissolved in dry dimethoxyethane (25 mL). Then while holding the temperature below 25°C, chlorosulfonyl isocyanate (3.08 g., 0.022 mmol) is added. After stirring overnight, the solution is treated with dimethylformamide (6 mL) and stirred for 3 hours. Finally, the solution is poured into water precipitating a brown solid (3.8 g, 90%). Dry column chromatography (80/20 hexane/ethyl acetate) yields 1.4 g (33%) of white solid with m.p. 202-204°C.

| | | | | | |
|---|---|---|---|---|---|
| Calcd for $C_{11}H_6BrClN_2$: | C, 46.90; | H, 2.13; | N, 9.95; | Cl, 12.61; | Br, 28.39. |
| Found: | C, 47.20; | H, 2.09; | N, 9.80; | Cl, 12.36; | Br, 27.42. |

## EXAMPLE 44

Preparation of 3,5-Dibromo-4-(p-chlorophenyl)pyrrole-2-carbonitrile

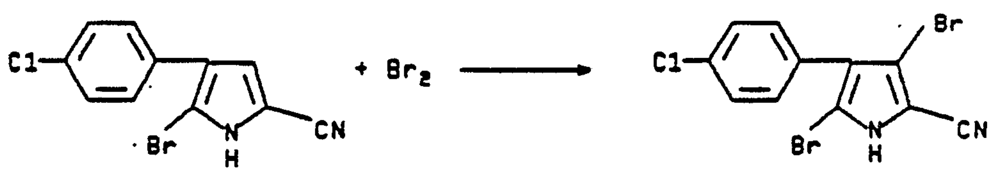


A sample of 5-bromo-4-(p-chlorophenyl)pyrrole-2-carbonitrile (2.2 g., 0.0078 mol) is dissolved in 30 mL of dry dioxane. The solution is heated with bromine (1.3 g., 0.008 mol) in dioxane (20 mL) and then stirred overnight at room temperature. The reaction mixture is poured into water precipitating a tan solid (2.6 g., 92%). A portion (1.6 g) is purified by flash chromatography using 75/25 hexane/ethyl acetate to give 0.8 g of grey solid with m.p. 191-194°C.

| | | | | | |
|---|---|---|---|---|---|
| Calcd for $C_{11}H_5Br_2ClN_2$: | C, 36.61; | H, 1.38; | N, 7.76; | Cl, 9.84; | Br, 44.3. |
| Found: | C, 37.46; | H, 1.25; | N, 7.41; | Cl, 9.53; | Br, 42.99. |

## EXAMPLE 45

Preparation of 3-(3,4-dichlorophenyl)-4-nitropyrrole

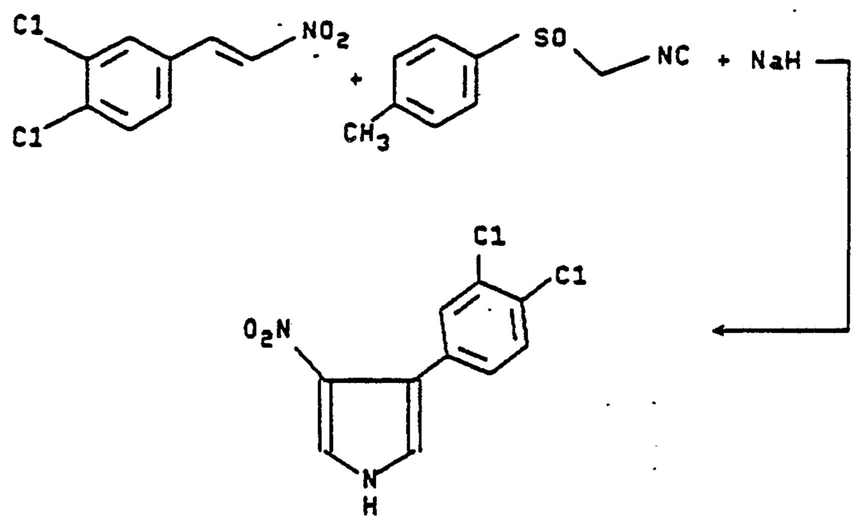

Sodium hydride (2.66 g of a 60% suspension in oil is rinsed with dry ether; 66 mmol) and suspended in 150 mL of dry ether. To this mixture is added over 15 minutes a mixture of 12.0 g (5.5 mmol) of 3,4-dichloro-β-nitrostyrene and 10.8 g (5.5 mmol) of (p-tolysulfonyl)methyl isocyanide in 50 mL of DMSO and 150 mL of ether. The mixture is stirred for 1.5 hours and then diluted with 150-200 mL of water and additional ether. The ether layer is separated, dried over magnesium sulfate, and concentrated *in vacuo*. The resulting 10.6 g of crude product is purified by chromatography on silica gel using a 4:1 mixture of chloroform and ethyl acetate. A 7.2 g solid fraction is recrystallized from chloroform-ethyl acetate-hexane to give 3.0 g of yellow solid, m.p. 187-188°C (dec.).

| | | | |
|---|---|---|---|
| Anal. Calcd for $C_{10}H_6Cl_2N_2O_2$: | C, 46.72; | H, 2.35; | N, 10.90. |
| Found: | C, 46.96; | H, 2.60; | N, 9.77 |

EXAMPLE 46

Preparation of 2,5-Dichloro-3-(3,4-dichlorophenyl)-4-nitropyrrole

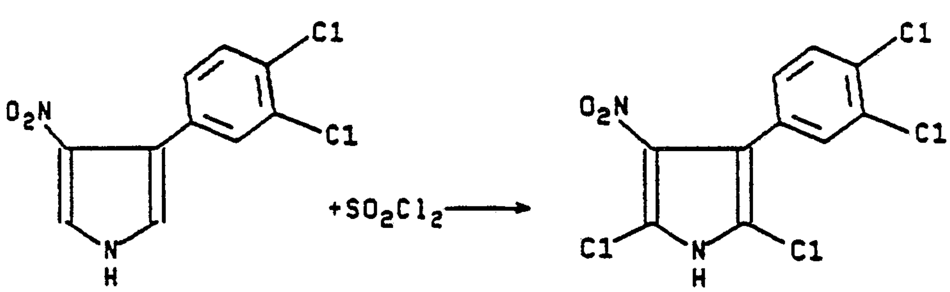


To a mixture of 3-(3,4-dichlorophenyl)-4-nitropyrrole (2.5 g, 9.7 mmol) warmed to about 40°C in 200 mL of chloroform is added over one minute 2.95 g (22 mmol) of sulfuryl chloride. After another hour, the mixture is diluted with 100 mL of saturated sodium bicarbonate solution and 300 mL of ether. The organic

layer is separated and dried over magnesium sulfate. Concentration, in vacuo, leaves a brown solid which is chromatographed on silica gel using 4:1 chloroform ethyl acetate. An orange solid fraction is recrystallized from chloroform and then rechromagraphed on silica gel using 4:1 chloroform ethyl acetate to yield 0.36 g of yellow solid, m.p. 193-194°C.

Also prepared by procedure of Examples 45 and 46 above is 2,5-dichloro-3-nitro-4-phenylpyrrole, m.p. 193-194°C(dec.).

## EXAMPLE 47

Preparation of 5-(p-chlorophenyl)pyrrole-2,4-dicarbonitrile

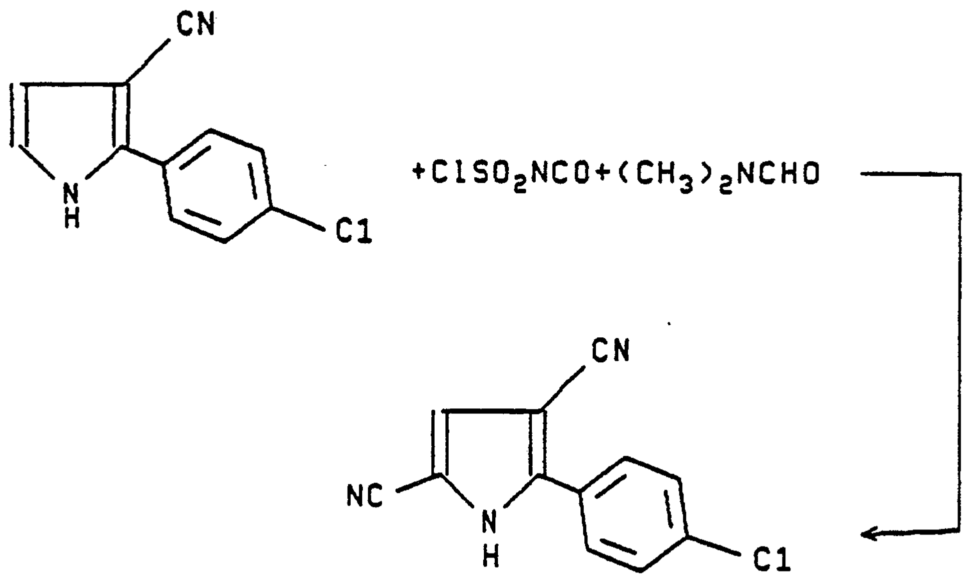


A sample of 2-p-chlorophenyl-3-cyanopyrrole, prepared by the method of Example 4, (3.0 g, 0.015 mole) is dissolved in 50 mL of dry dimethoxyethane. To this solution is added chlorosulfonyl isocyanate (3.39 g, 0.024 mole). The addition is exothermic and some cooling is necessary. After stirring 3 hours at room temperature, dimethylformamide (6-7 mL) is added and the solution is stirred 4 hours more. The solution is then poured into water precipitating a white solid (3.4 g, 100%). A sample (1.0 g) is purified by dissolving in ethyl acetate and then passing the solution through a 60 mL course filter funnel packed with silica gel. The filtrate is concentrated to yield 0.7 g of a white solid with m.p. 235-240°C.

Following the procedure of Example 47, the following analogs are prepared:

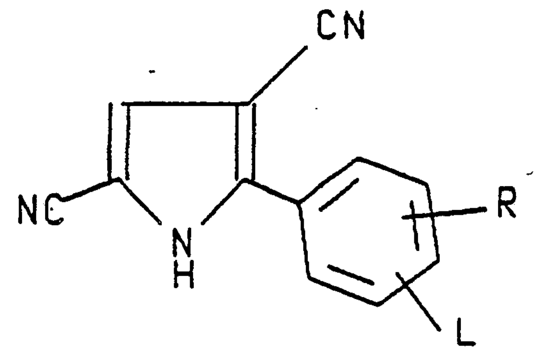

| R | L | m.p. |
|---|---|---|
| 3-Cl | 4-Cl | >225°C |
| H | $4-OCF_3$ | 185-190°C |
| H | $4-CF_3$ | 180-185°C |

## EXAMPLE 48

Preparation of 3-Bromo-5-(p-chlorophenyl)pyrrole-2,4-dicarbonitrile

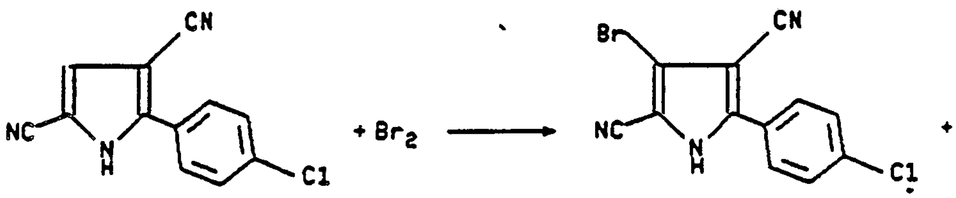


A sample of 5-(p-chlorophenyl)pyrrole-2,4-dicarbonitrile (1.0 g, 0.004 mole) is dissolved in 20 mL of dioxane and a solution of bromine (0.8 g, 0.005 mole) in dioxane (10 mL) is then added thereto. The solution is stirred several hours at room temperature and then poured into water precipitating a white solid (1.2 g, 100%). The solid has a m.p. >225°C and a mass spectrum of a sample gives a pattern consistent with the desired structure.

Following the procedure set forth above in Example 48, the following additional compounds are prepared:

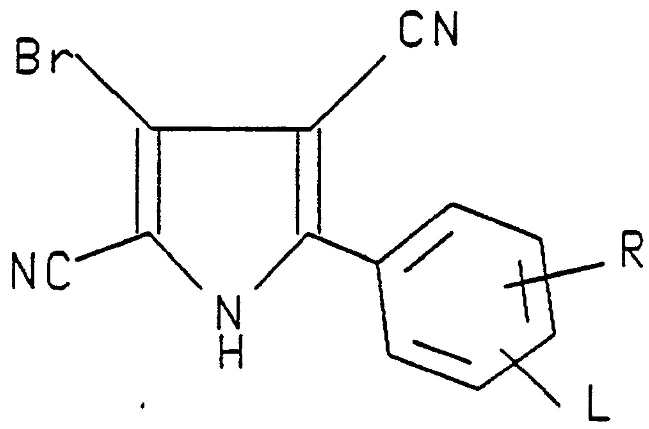

| **R** | **L** | **m.p.** |
|---|---|---|
| 3-Cl | 4-Cl | >250°C |
| H | 4-$OCF_3$ | 218-223°C |
| H | 4-$CF_3$ | 239-241°C |

## EXAMPLE 49

Preparation of bromofumaronitrile

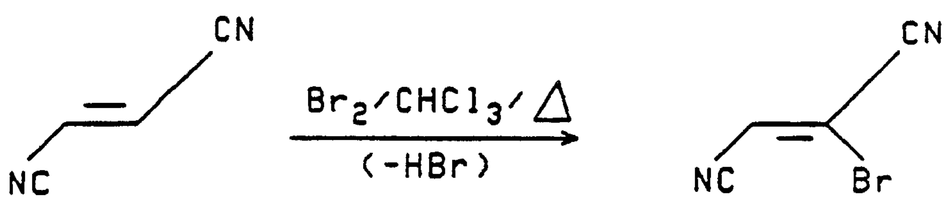


Under a nitrogen purge, fumaronitrile (15.6 g; 0.2 mol) in $CHCl_3$ (150 mL) is heated to reflux, resulting in a clear solution. A solution of bromine (5.3 mL; 0.2 mol) in $CHCl_3$ (25 mL) is added dropwise over 30 minutes, resulting in a slow decolorization and acidic (pH test paper) fumes being released. The solution is refluxed another 90 minutes, during which time most of the color has been discharged. The solution is cooled and solvent is removed under reduced pressure, leaving an amber oil (weight approximately theoretical for bromofumaronitrile). The oil is subjected to bulb-to-bulb distillation (0.2 mm Hg), maintaining the temperature below 120°C (above that point, a rapid decomposition of material occurs). A semi-solid is obtained which slowly forms a waxy, amber solid, m.p. - 43-47°C.

| | | | |
|---|---|---|---|
| Calcd for $C_4HBrN$: | C, 30.57; | H, 0.64; | N, 17.83. |
| Found: | C, 29.13; | H, 0.75; | N, 16.94. |

EXAMPLE 50

Preparation of 2-phenyl-pyrrole-3,4-dicarbonitrile

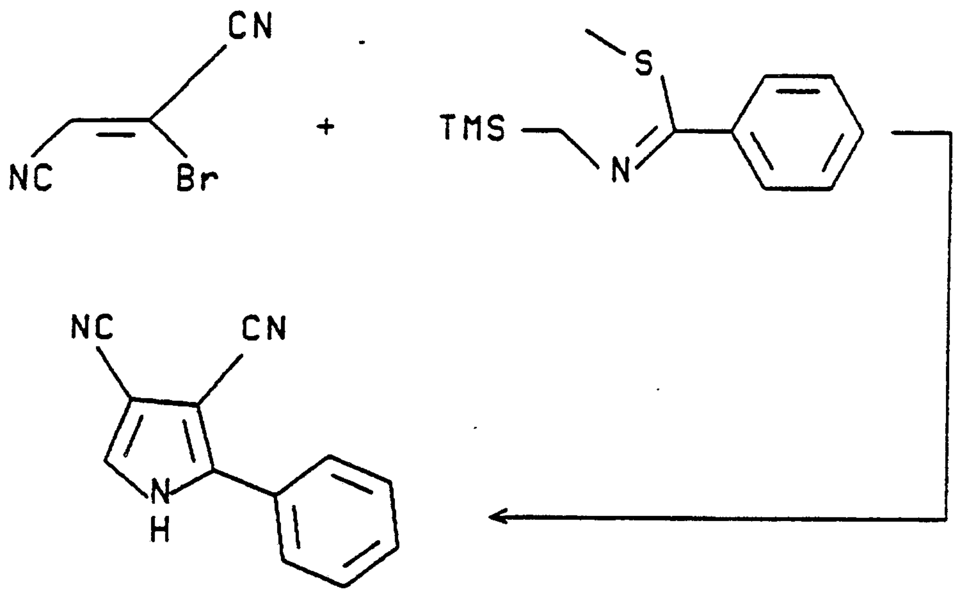

Under a nitrogen purge, a solution of bromofumaronitrile (4.7 g; 0.03 mol) and N-(trimethylsilyl)methyl-S-methyl-benzene-thioimidate (7.1 g; 0.03 mol) in hexamethylphosphoramide (HMPA) (35 mL) is stirred at room temperature. In a single portion, water (1.6 mL); 0.09 mol) is added, washed in with HMPA (10 mL). The solution almost immediately begins to exotherm, the temperature rapidly reaching 100°C before subsiding. The resulting dark red solution is allowed to stir at ambient temperature 20 hours. Pouring the reaction mixture onto an ice/water mixture results in a gummy material which slowly yields a discreet beige solid. This material is collected by filtration and washed with cold water and dried on the filter. After further drying (vacuum oven; 60°C), the material is twice recrystallized from $C_2H_4Cl_2$ (DARCO treatment) to yield a white powder.

| | | | |
|---|---|---|---|
| Calcd for $C_{12}H_7N_3$: | C, 74.61; | H, 3.63; | N, 21.76. |
| Found: | C, 74.45; | H, 3.84; | N, 21.61. m.p. = 197-200°C. |

EXAMPLE 51

Preparation of 2-bromo-5-phenylpyrrole-3,4-dicarbonitrile

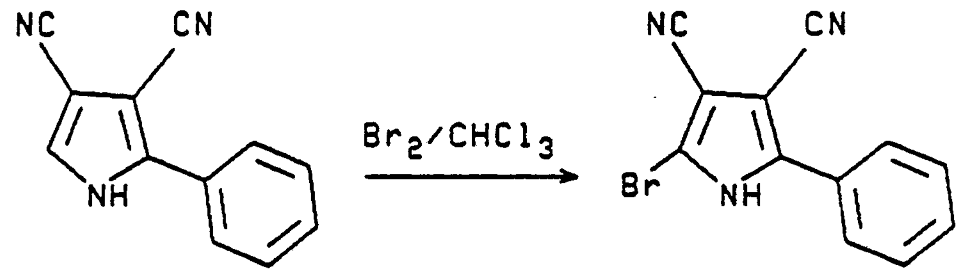


Under a nitrogen purge, 2-phenyl-pyrrole-3,4-dicarbonitrile (1.4 g; 0.0075 mol) is added to $CHCl_3$ (35 mL), much of the solid dissolving. A solution of bromine (0.4 mL; 0.008 mol) in $CHCl_3$ (5 mL) is added dropwise over 20 minutes. Initially the color is discharged rapidly, but as a new gummy solid begins to precipitate, the color remains. After stirring 30 minutes at ambient, the mixture is brought to reflux, resulting in a much more discreet solid. After refluxing 90 minutes, the reaction mixture is cooled and an aliquot is removed and analyzed (HPLC), showing ca. 60% starting material still remaining. In a single portion fresh bromine (0.2 mL; 0.004 mol) is added, and refluxing continued another 45 minutes whereupon an aliquot shows 10% starting material remaining. Another fresh portion of bromine (0.2 mL; 0.004 mol) is added to the refluxing suspension and refluxing is continued another 30 minutes. The suspension is cooled and stirred 18 hours at room temperature. Solvent is removed under reduced pressure to yield a greenish solid which is extracted with hot $CHCl_3$, leaving behind a dark residue. The extract is treated with DARCO and filtered hot. The clear yellow filtrate quickly began to deposit a white precipitate. After cooling to -10°C, the white solid is collected by filtration.

| | | | | |
|---|---|---|---|---|
| Calcd for $C_{12}H_6BrN_3$: | C, 52.94; | H, 2.21; | N, 15.44; | Br, 29.41. |
| Found: | C, 51.64; | H, 2.35; | N, 14.91; | Br, 28.69. m.p. = 225-258°C. |

## EXAMPLE 52

Preparation of 2-(3,4-Dichlorophenyl)-5-nitropyrrole-3-carbonitrile

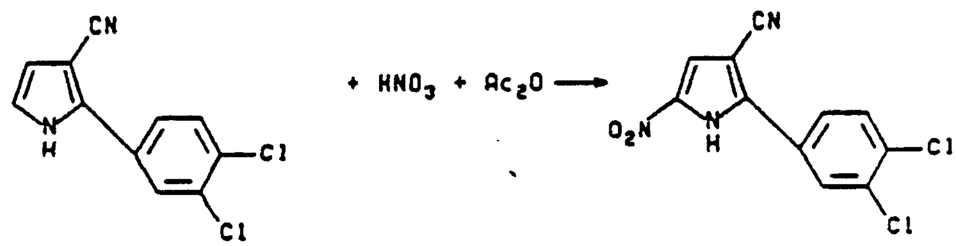


2-(3,4-Dichlorophenyl)pyrrole-3-carbonitrile (3.0g, 0.013 mole) is added to acetic anhydride (50 mL) and 90% nitric acid (0.6 ml) with very little exotherm. The mixture is slowly warmed to 30° and is then held at 30-33° until everything goes into solution. Gradually a new solid precipitates. The mixture is stirred for 2 to 3 hours at room temperature and then poured into water and ice to decompose the acetic anhydride. After stirring 1 hour the mixture is filtered and the solid (2.9 g, 82%) collected and dried. A portion (1.5 g) is purified by column chromatography on silica gel using 75/25 hexane/ethyl acetate for elution to give 0.7 g of yellow solid with m.p. 228-231°.

| | | | | |
|---|---|---|---|---|
| Calcd for $C_{11}H_5Cl_2N_3O_2$: | C, 46.80; | H, 1.77; | N, 14.89; | Cl, 25.17 |
| Found: | C, 46.50; | N, 1.96; | N, 14.27; | Cl, 24.30. |

By the same procedure, starting with 2-(p-chlorophenyl)pyrrole-3-carbonitrile, 2-(p-chlorophenyl)-5-nitropyrrole-3-carbonitrile is obtained, m.p. 201-206°C. Also, 2-(p-trifluoromethylphenyl)pyrrole-3-carbonitrile gives 2-(p-trifluoromethylphenyl)-5-nitropyrrole-3-carbonitrile by the above procedure. Ths compound has a melting point of 164-165.5°C.

## EXAMPLE 53

Preparation of 4-Bromo-2-(3,4-dichlorophenyl)-5-nitropyrrole-3-carbonitrile

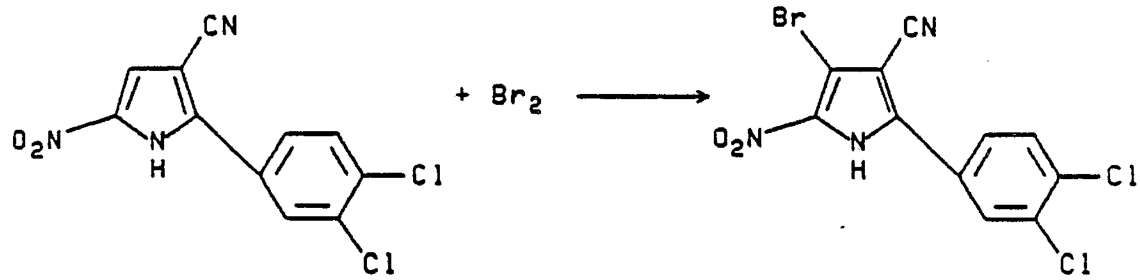


2-(3,4-Dichlorophenyl)-5-nitropyrrole-3-carbonitrile (0.5 g, 0.0017 mol) is dissolved in dry dioxane (10 mL). To this solution is added bromine (0.28 g, 0.0017 mole) in dioxane. After stirring overnight, the solution is poured into water precipitating a tan solid (0.54 g, 88%). Recrystallization from acetonitrile (5 mL) gives 0.26 g of tan solid with m.p. 195-200°C.

| | | | | | |
|---|---|---|---|---|---|
| Calcd for $C_{11}H_4BrCl_2N_3O_2$: | C, 36.57; | H, 1.10; | N, 11.63; | Br, 22.13; | Cl, 19.67. |
| Found: | C, 36.46; | H, 1.29; | N, 11.50; | Br, 21.63; | Cl, 19.28. |

Following the above procedure of Example 53, but starting with 2-(p-chlorophenyl)-5-nitropyrrole-3-carbonitrile gives 4-bromo-2-(p-chlorophenyl)-5-nitropyrrole-3-carbonitrile, m.p. 180-185°C.

## EXAMPLE 54

5-(3,4-Dichlorophenyl)-4-nitropyrrole-2-carbonitrile

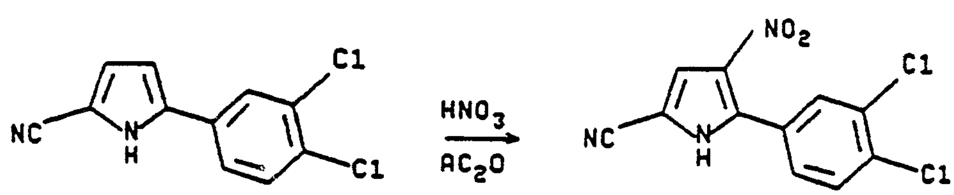

To a suspension of 5-(3,4-dichlorophenyl)pyrrole-2-carbonitrile (1,2 g, 5.1 mmol) in 25 mL of acetic anhydride at 30° under nitrogen, is added dropwise 90% nitric acid (0.3 mL, 5.1 mmol). The reaction exotherms to 45°C and becomes a green solution. After being allowed to stir for 2 hours the reaction is poured into 50 mL of water and stirred vigorously for 5 minutes. The beige precipitate which results is filtered off and dissolved in a minimum amount of acetone. Chromatography over silica gel using 3:1 hexane-ethyl acetate affords the nitropyrrole (1.2 g, 84%) as an off-white solid, m.p. >200°C.

## EXAMPLE 55

3-Bromo-5-(3,4-dichlorophenyl)-4-nitropyrrole-2-carbonitrile

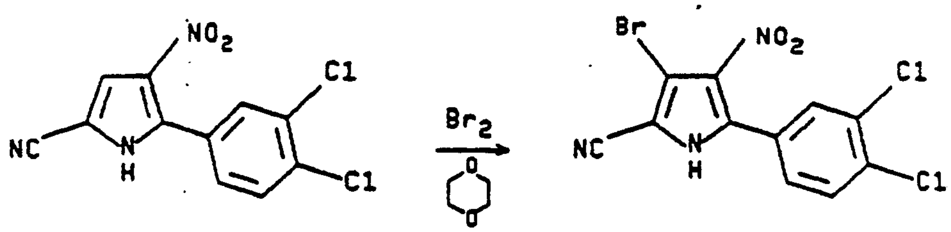


To a suspension of 5-(3,4-dichlorophenyl)-4-nitropyrrole-2-carbonitrile (0.6 g, 2.1 mmol) in 10 mL of dioxane at 25°C, under nitrogen, is added dropwise a solution of bromine (0.3 g, 2.1 mmol) in 5 mL of dioxane. The reaction is allowed to stir overnight. Addition of 50 mL of water causes precipitation of a yellow solid which is collected and vacuum oven dried (50 mm Hg, 45°C) to afford thebrominated pyrrole (0.7 g, 90%) as a light yellow solid, m.p. >200°C.

## EXAMPLE 56

4-(p-chlorophenyl)-2-(trifluoromethyl-2-oxazolin-5-one

In a single portion, trifluoroacetic anhydride, (1.7 mL; 0.012 mol) is added to powdered 2-(p-chlorophenyl)glycine (11.4 g; 0.06 mol), causing an immediate exotherm to about 40°C, a yellow color forming on the surface of the solid. As the mixture is slowly heated to 70°C, more of the solid dissolves to an orange/amber oil. All the solid dissolved in approximately 2 hours, and heating is continued another hour. Solvent is removed under reduced pressure on a rotary evaporator. Toluene is twice added and removed under reduced pressure, but the odor of trifluoroacetic acid is still evident. This yellow semi-solid (yield theorectical; purity > 90% by HPLC) is the above-identified compound and is used in the next step without further purification.

## EXAMPLE 57

Preparation of 2-(p-chlorophenyl)-5-(trifluoromethyl) pyrrole-3-carbonitrile

4-(p-chlorophenyl)-2-(trifluoromethyl)-2-oxazolin-5-one (2.5 g; 0:01 mol) is dissolved in nitromethane (50 mL). In a single portion, 2-chloroacrylonitrile (8.0 mL; 0.10 mol) is added to the solution, and the resulting solution is stirred 18 hours at reflux under a nitrogen atmosphere. Cooling the red/brown solution to -5°C in an ice-acetone bath causes the formation of a precipitate which is collected by filtration and washed with a small portion of cold nitromethane. The resulting tan solid is recrystallized from hot ethylene dichloride

yielding the product as white crystals (1.8 g; 56% theory), m.p. 238-241°C (dec.).

By utilizing the appropriate arylglycine in the procedure of Example 55 and following the procedure of this Example, the following 2-aryl-5-(trifluoromethyl)pyrrole-3-carbonitrile were prepared:

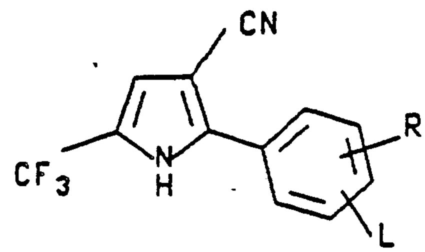

| R | L | m.p. °C |
|---|---|---|
| H | H | 215-218 |
| H | 4-$CH_3$ | 191-193 |
| H | 4-$OCH_3$ | 168-180(dec.) |
| 3-Cl | 4-Cl | 245-246(dec.) |
| H | 4-$CF_3$ | 218-219 |

## EXAMPLE 58

Preparation of 4-Bromo-2-(p-chlorophenyl)-5-(trifluoromethyl)pyrrole-3-carbonitrile

Under a nitrogen purge, a suspension or 2-(p-chlorophenyl)-5-(trifluoromethyl)pyrrole-3-carbonitrile (1.6 g; 0.005 mol) in acetic acid (25 mL) is heated, all the material dissolving to a clear solution at about 60°C. A solution of bromine (0.8 mL; 0.015 mol) in acetic acid (10 mL) is added dropwise over 15 minutes to the refluxing solution. The solution is refluxed 6 hours then allowed to stir 18 hours at room temperature. The HPLC of the reaction mixture shows about 80% conversion to product. The mixture is heated back to reflux and more bromine (0.5 mL; 0.01 mol) in acetic acid (5 mL) is added dropwise. After refluxing another 3 hours, the aliquot shows > 95% conversion to product. The reaction is cooled, and solvent removed under reduced pressure on a rotary evaporator to obtain a dark grey solid. Toluene is added to the mixture and removed under reduced pressure, but the odor of acetic acid still remains. The entire material is dissolved in hot toluene (75 mL) to a turbid solution which is treated with DARCO filter and filtered. The light pink solution deposits a white solid upon cooling to ambient. After cooling in the freezer, the solid is collected by filtration, washed with hexanes, and dried on the filter. Further drying in a vacuum oven at 45°C provides the product (1.2 g; app. 60% theorectical); m.p. 247-250°(dec.).

| | | | | | | |
|---|---|---|---|---|---|---|
| Anal. Calcd for $C_{12}H_5BrClF_3N_2$: | C, 41.20; | H, 1.43; | N, 8.01; | Br, 22.89; | Cl, 10.16; | F, 16.31. |
| Found: | C, 41.27; | H, 1.48; | N, 8.10; | Br, 22.92; | Cl, 10.16; | F, 16.03. |

By brominating the appropriate 2-aryl-5-)(trifluoromethyl)pyrrole-3-carbonitrile, obtained by the procedure of Example 57, according to the above recipe, the following additional examples are prepared:

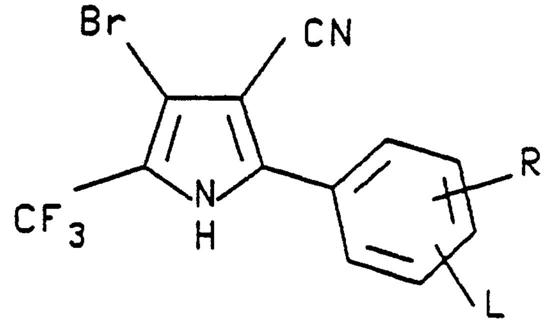

| R | L | m.p. °C |
|---|---|---|
| H | H | 235-238 |
| H | 4-$CH_3$ | 244-245 |
| 3-Cl | 4-Cl | 218-223 |
| H | 4-$CF_3$ | 225-226 |

EXAMPLE 59

Preparation of 2-(4-chlorophenyl)-5-trifluoromethylpyrrole-3,4-dicarbonitrile

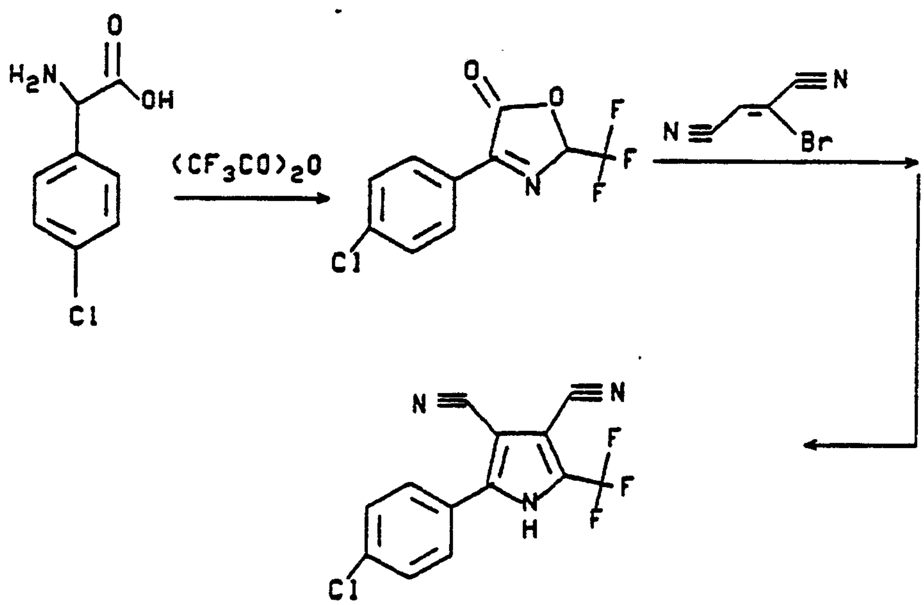


Trifluoroacetic anhydride (3.1 mL; 0.022 mol) is added in a single portion to (4-chlorophenyl)glycine (2.0 g; 0.011 mol), causing an immediate yellow color and some refluxing. The mixture is slowly heated to reflux, causing all the material to dissolve to a yellow/orange solution which is heated 2 hours further. The reaction mixture is cooled, and solvent removed under reduced pressure. Toluene, is twice added and removed under reduced pressure to yield a very thick oil ($V_{co}$ = 1800 $cm^{-1}$). This residue is dissolved (some insolubles) in $CH_3NO_2$ (40 mL) and bromofumaronitrile (2.7 g; 0.018 mol) is added in a single

portion. The resulting solution is heated at reflux 18 hours, yielding a dark red solution. Solvent is removed under reduced pressure and the dark residue is dissolved in $CH_2Cl_2$, some insolubles being removed by filtration. The material is fractionated via dry column chromatography (silica gel; 3% 2-PrOH in $CH_2Cl_2$), and appropriate fractions are taken. Evaporation of one fraction yields the desired compound as a yellow solid which is recrystallized from $CH_3CN$ (DARCO treatment) to yield a pale yellow solid (0.2 g). m.p. = 238-241°C. (some dec).

## EXAMPLE 60

### Evaluation of the fungicidal activity of compounds of the invention

#### In Vivo

To determine the effectiveness of the arylpyrrole compounds as fungicidal agents, a variety of pathogenic fungi, host plants, and arylpyrrole compounds are used in the following tests. Pathogens, host plants, the method of testing, and the rating system used are reported below, along with the data so obtained.

### General Procedure For The *In Vivo* Fungicide Screen

A 40 mg sample of a test compound is dissolved in 5 mL of acetone and diluted with 95 mL deionized water containing 0.05% TWEEN 20®, a polyoxyethylene sorbitan monolaurate surfactant of Atlas Chemical Industries, to give a preparation containing 400 ppm of the test compound. Lower concentrations are obtained by diluting this formulation with 0.05% TWEEN 20®. If a test compound is insoluble in both an organic solvent and water, it is prepared as a fine suspension ultrasonically or with a glass tissue grinder.

Formulated test compounds are sprayed on the host plants so as to produce a fine film of spray droplets on all foliar surfaces. This is accomplished using a linear spray chamber in which plants on a moving belt pass through a spray pattern formed by four nozzles in fixed positions above and below the plants.

After drying, the plants are inoculated with fungi that cause the following diseases.

| Disease | Pathogen |
|---|---|
| RB = Rice Blast | *Pyricularia oryzae* |
| WLR = Wheat Leaf Rust | *Puccinia recondita tritici* |
| CDM = Cucumber Downy Mildew | *Pseudoperonospora cubensis* |
| TLB = Tomato Late Blight | *Phytophthora infestans* |
| AS = Apple Scab | *Venturia inaequalis* |
| GDM = Grape Downy Mildew | *Plasmopara viticola* |
| WPM = Wheat Powdery Mildew | *Erysiphe graminis* |

Rice Blast Protective Test

Rice seedlings (variety M9) are grown in 2 1/2 inch square plastic pots using Metro Mix 350, a mixture of Canadian sphagnum peat moss, domestic horticultural vermiculite, processed ash bark and washed granite sand manufactured by Grace Horticultural Products. After 12-14 days, the rice seedlings are sprayed with the test compound as described above and approximately four hours later, the foliage is inoculated with a spore suspension of the rice blast fungus using a DeVilbiss atomizing sprayer. The inoculated plants are placed in a dew chamber for 24 hours at room temperature to initiate infection, then

moved to a greenhouse until disease symptoms appear, about 7-10 days later.

The spore suspension is prepared by scraping spores from a 10-12 day old culture of Pyricularia oryzae growing on rice polish agar (20 g rice polish, and 17 g agar and 1L deionized water) into deionized water containing 0.05% TWEEN 20®, filtering through four layers of cheese cloth, and adjusting the concentration to from 55% to 60% light transmission at 450 ηm on a Bausch and Lomb Spectronic 20 colorimeter.

### Wheat Leaf Rust Protective Test

Wheat seedlings (variety Hart), grown in two and one-half inch plastic pots in Metro Mix 350 for seven days, are treated with the test compound as described hereinabove and approximately four hours later are inoculated with a spore suspension of the leaf rust fungus using a DeVilbiss atomizing sprayer. The inoculated plants are placed in a dew chamber for 24 hours at room temperature to initiate infection, then moved to a greenhouse until disease symptoms appear, about 7-10 days later.

The spore suspension is prepared by harvesting spores from greenhouse plants infected with Puccinia recondita tritici and suspending them in deionized water containing 0.1% TWEEN 20® at a concentration of 2 mg spores/mL.

### Cucumber Downy Mildew Protective Test

Cucumber plants (variety Straight 8) grown in three inch plastic pots in Metro Mix 350 for 12-14 days, are treated with the test compound as described hereinabove and after approximately four hours, the foliage is inoculated with a sporangial suspension of the downy mildew fungus using a DeVilbiss atomizing sprayer. The inoculated plants are placed in a dew chamber for 24 hours at room temperature to initiate infection, then moved to greehouse benches until disease symptoms appear, about 5-7 days later.

The sporangial suspension is prepared by washing sporangia from the under side of cucumber leaves infected with Pseudoperonospora cubensis into deionized water. The suspension is visibly cloudy.

### Tomato Late Blight Protective Test

Tomato plants (variety Rutgers) grown in three inch plastic pots in metro Mix 350 for four weeks, are treated with the test compound as described hereinabove and after approximately four hours, the foliage is inoculated with a zoospore/sporangial suspension of the tomato late blight fungus using a DeVilbiss atomizing sprayer. The inoculated plants are placed in a dew chamber for 24 hours at room temperature to initiate infection, then moved to a 70°/60°F (day/night) growth chamber with 12 hours day length and high humidity until disease symptoms appear, about 5-7 days later.

The spore suspension is prepared by washing sporangia from a 12 day old culture of Phytophthora infestans growing on a V-8® juice agar (200 mL V-8® juice, 15 g agar, $CaCO_3$ to pH7, and 1L deionized water) into glass distilled water and adjusting to 50,000 sporgangia/mL. This suspension is placed at 15°C for five hours to induce zoospore production, and then used for inoculation.

### Apple Scab Protective Test

Apple seeds are germenated in a flat containing perlite and vermiculate and stored in a cold room for 7 weeks following the vernalization period maintained at greenhouse for 1 week and then transplanted to 3 inch square plaster pots containing metro-mix 350. After 12-14 days the apple seedlings are sprayed with the test compound as described above and approximately four hours later the foliage is inoculated with a conidial suspension of the apple scab fungus using a DeVilbiss atomizing sprayer. The conidial suspension is prepared thusly: Infected seedlings are cut off and then shaken in a solution of 0.05% TWEEN 20 in deionized water in a glass jar. The resulting conidial suspension is filtered through 8 single-layers of cheesecloth and then diluted with 0.05% TWEEN 20 water to 1,000,000 conidia per ml as determined by a hemocytometer.

**Grape Downy Mildew Protective Test**

Individual dormant canes, 1-3 inches long with 1 node each, are planted in Jiffy-7 peat pellets and placed in the greenhouse on heating mats for rooting. After 3-4 weeks the rooted cuttings are transplanted into 3" pots containing Metro-mix 350. Plants are ready for testing 3-4 weeks later when 2-3 leaves have expanded sufficient to allow for treatment and inoculation. Formulated test compounds are applied to all foliar surface to the point of run-off. Approximately four hours later hand-held DeVilbiss atomizer is used to inoculate the foliage with a sporangial suspension of the grape downy mildew fungus, targeting the undersides of the leaves. Inoculated plants are placed in a dew chamber for 48 hours. Disease control is evaluated about 12 days after inoculation.

The sproangial suspension is prepared as follows: On the day before inoculation, grape plants infected with Plasmopara viticola are placed in a moist chamber to induce sporulation. On inoculation day, leaves with sporangia are shaken in deionized water in a glass jar. The resulting sporangial suspension is filtered through 8 single-layers of cheesecloth and diluted with deionized water to 20,000 sporangia per ml as determined by a hemacytometer.

**Wheat Powdery Mildew Protective Test**

Wheat seed is grown in 2.25-inch square plastic pots containing Metro-mix 350. Approximately one week after seeding formulated test compounds are applied to all foliar surfaces. After about four hours the wheat plants are inoculated by dusting them with conidia from powdery mildew infected wheat plants. Disease evaluations are made about a week after inoculation.

The effectiveness of test compounds in controlling disease is rated on a 0-9 scale defined below.

| Rating | Range % Control |
|---|---|
| 0 | 0 |
| 1 | 0 - 19 |
| 2 | 20 - 29 |
| 3 | 30 - 39 |
| 4 | 40 - 59 |
| 5 | 60 - 74 |
| 6 | 75 - 89 |
| 7 | 90 - 96 |
| 8 | 97 - 99 |
| 9 | 100 |
| - | no evaluation |

Phytotoxicity is rated on a 1-5 scale defined below.

| Rating | Injury |
|---|---|
| 1 | trace |
| 2 | slight |
| 3 | moderate |
| 4 | severe |
| 5 | death |

Letters are used to denote type of injury such as:

N = necrosis
S = stunting
Sc = scorching
F = formative/curling

The data obtained is shown in Table III, III A, III B and III C below.

## TABLE III

### Evaluation of 2-Arylpyrrole Compounds For The Control of Plant Pathogenic Fungi

| Compound | Rate (ppm) | Disease Control/Phytotoxicity TLB | RB | CDM | WLR |
|---|---|---|---|---|---|
| 4,5-Dichloro | 400 | 9/4N | 5/4N | -/5 | 9/3N |
| -2-(p-chloro- | 100 | 8/3N | 0/3N | 9/4N | 9/2N |
| phenyl)pyrrole | 25 | 3 | 0/2N | 6/2N | 8/2N |
| -3-carbonitrile | 12.5 | - | - | - | 8/1S |
| | 6.25 | - | - | - | 7/1S |
| 2,3-Dichloro-5- | 400 | 7/4N | 9/3N | -/5 | 9/3N |
| (p-chlorophenyl) | 100 | 7/2N | 8/2N | 8/3N | 8/2N |
| -4-nitropyrrole | 25 | 6/1N | 8/2N | 7/1N | 8/1N |
| 2,3-Dichloro-5 | 100 | 9/3Sc | 3/1Sc | 9/3N | - |
| -(p-chlorophenyl) | 25 | 8/1N | - | 8 | - |
| -4-nitropyrrole | 6.25 | 0 | - | 6 | - |
| 2-(p-Bromo- | 100 | 9/3Sc | 5/3Sc | 8/1N | - |
| phenyl)-4,5- | 25 | 9/1N | - | 8/1N | - |
| dichloro-3- | 6.25 | 0 | - | 8 | - |
| nitropyrrole | | | | | |
| 2,3-dichloro | 200 | -/5 | 6/4N | -/5 | 9/2N |
| -4-nitro-5- | 50 | 8/3N | 8/3N | 6/2N | 9/1N |
| (α,α,α-tri- | 12.5 | 0/1N | 8/2N | 3 | 8 |
| fluoro-p- | | | | | |
| tolyl)pyrrole | | | | | |

**TABLE III Continued)**

| Compound | Rate (ppm) | Disease Control/Phytotoxicity TLB | RB | CDM | WLR |
|---|---|---|---|---|---|
| 2-(o-Chloro-phenyl)pyrrole-3-carbonitrile | 400 | 6 | 7/1N | 7 | 9 |
| | 200 | - | 8 | 6 | 0 |
| | 50 | - | 2 | 1 | 0 |
| | 12.5 | - | 0 | 1 | 0 |
| 4,5-Dibromo-2-(o-chlorophenyl)-pyrrole-3-carbonitrile | 400 | 3 | 7/1N | 9/3N | 9 |
| | 200 | - | 0 | 7/1F | 8 |
| | 50 | - | 0 | 2 | 9 |
| | 12.5 | - | 0 | 1 | 1 |
| 4,5-Dichloro-(α,α,α-tri-fluoro-m-tolyl)-pyrrole-3-carbonitrile | 400 | -/5N | 9/4N | -/5N | 9/3N |
| | 100 | 5/1N | - | 8 | 6/1N |
| | 25 | 0 | - | 5 | 4/1N |
| | 6.25 | 0 | - | 0 | 2 |
| 4,5-Dichloro-2-(3,5-dichloro-phenyl)pyrrole-3-carbonitrile | 400 | - | - | 9 | 6 |
| | 100 | - | - | 8 | 4 |
| | 25 | - | - | 7 | 2 |
| 4,5 Dichloro-2-(o-chlorophenyl)pyrrole-3-carbonitrile | 400 | 3 | 7/1N | 9/3N | 9 |

**TABLE III Continued)**

| Compound | Rate (ppm) | Disease Control/Phytotoxicity TLB | RB | CDM | WLR |
|---|---|---|---|---|---|
| 2,3-dibromo-4-nitro-5-phenyl pyrrole | 100 | 9/2N | | | |
| 2,3-dibromo-5-(p-chlorophenyl)-4-nitropyrrole | 100 | 9/2N | | | |
| 4-bromo-2-phenyl-5(trifluoromethyl) pyrrole-3-carbonitrile | 400 | 9/2N | 9/3N | | 9/1N |
| | 100 | | 7/1N | | |
| | 100 | 8/3N | 9/2N | | 5 |
| | 25 | | 9/1N | | 5 |
| | 25 | 4/2N | 5/1N | | |
| 4,5-dichloro-2-(p-chlorophenyl) pyrrole-3-carbonitrile | 100 | | | | 9/2N |
| 4-bromo-2-(p-chlorophenyl)-5-nitropyrrole-3-carbonitrile | 25 | | | | 9 |
| 3-bromo-5-(p-chlorophenyl) pyrrole-2,4-dicarbonitrile | 25 | | | | 9/2N |

## TABLE III Continued)

| Compound | Rate (ppm) | Disease Control/Phytotoxicity TLB | RB | CDM | WLR |
|---|---|---|---|---|---|
| 2-(3,5-difluoro- | 400 | | 7 | | 9/2N |
| phenyl)-5-(tri- | 400 | | 9/1N | | |
| fluoromethyl) | 100 | | 7/2N | | 9/2N |
| pyrrole-3- | 25 | | 5 | | 8/1N |
| carbonitrile | | | | | |
| 5-(trifluoromethyl) | 400 | | 8 | | 8/1N |
| -2-(α,α,α-tri- | 400 | | 9/4N | | |
| fluoro-p-tolyl) | 100 | | 4 | | 8/1N |
| pyrrole-3- | 25 | | 5 | | 6 |
| carbonitrile | | | | | |

## TABLE III A

### Evaluation of Arylpyrrole Compounds for The Control of Apple Scab

| Compound | Rate | Disease Control/Phytotoxicity |
|---|---|---|
| 1-methyl-5-(α,α,α-trifluoro-p-tolyl)pyrrole-2,4-dicarbonitrile | 100 | 9 |
| | 25 | 9/2N |
| 5-bromo-1-(ethoxymethyl)-2-(α,α,α-trifluoro-p-tolyl pyrrole-3-carbonitrile | 100 | 9/2N |

TABLE III B

Evaluation of Arylpyrrole for
The Control of Grape Downy Mildew

| Compound | Rate | Disease Control/Phytotoxicity |
|---|---|---|
| 2,3-dichloro-4-nitro-5-phenylpyrrole | 100 | 9/2N |
| 2,3-dibromo-5-(p-chloro phenyl)-4-nitropyrrole | 100 | 9/2N |
| 2-(p-bromo-phenyl)-4,5-dichloro-3-nitropyrrole | 100 | 9/1N |
| 4,5-dichloro-2-(m-chlorophenyl) pyrrole-3-carbonitrile | 100 | 9/1N |
| 2,5-dibromo-3-nitro-4-phenyl-pyrrole | 100 | 9 |

## TABLE III C

### Evaluation of Arylpyrrole for Control of Wheat Powdery Mildew

| Compound | Rate | Disease Control/Phytotoxicity |
|---|---|---|
| 4-bromo-2-(*o*-fluorophenyl-5-(trifluoromethyl)pyrrole-3-carbonitrile | 100 | 9 |
| 4-bromo-1-(ethoxymethyl)-2-(*o*-fluorophenyl)-5-(trifluoromethyl)-pyrrole-3-carbonitrile | 100 | 9 |
| | 25 | 9 |
| 4-bromo-2-(3,5-difluorophenyl)-1-(ethoxymethyl)-5-(trifluoromethyl) pyrrole-3-carbonitrile | 400 | 9 |
| | 400 | 9/1N |
| | 100 | 9/1N |
| | 25 | 7 |
| 4,5-dichloro-2-(3,4-dichlorophenyl)-1-(1-methoxyethyl) pyrrole-3-carbonitrile | 100 | 9 |

TABLE III C Continued

Evaluation of Arylpyrrole for Control of Wheat Powdery Mildew

| Compound | Rate | Disease Control/Phytotoxicity |
|---|---|---|
| 3-nitro-2-phenyl-4,5-bis(trifluoromethyl)pyrrole | 100 | 9/1N |
| 4,5-dichloro-2-(3,4-dichlorophenyl)-1-(1-isopropoxyethyl)pyrrole-3-carbonitrile | 400 | 9 |
| | 400 | 9 |
| | 100 | 9 |
| | 25 | 9 |
| 4,5-dichloro-2-(p-chlorophenyl)-1-(isopropoxymethyl)pyrrole-3-carbonitrile | 400 | 8 |
| | 400 | 9 |
| | 100 | 9 |
| | 25 | 9 |
| 4-bromo-5-(trifluoromethyl)-2-(α,α,α-trifluoro-p-tolyl)pyrrole-3-carbonitrile | 100 | 7/3N |
| | 25 | 7/2N |
| 4-bromo-2-(p-chlorophenyl)-1-(ethoxymethyl)-5-(trifluoromethyl)pyrrole-3-carbonitrile | 400 | 7 |
| | 400 | 9/1N |
| | 100 | 7 |
| | 25 | 7 |

## EXAMPLE 61

Evaluation of the inhibition of the mycelial growth of selected plant pathogenic fungi by compounds of the invention

### In Vitro

Severe soilborne diseases such as damping off, seedling blight, crown rot, and root rot, among others are caused by certain plant pathogenic fungi. An in vitro assay is conducted to determine whether test compounds will inhibit the mycelial growth of said fungi. Pathogens, the method of testing, and the rating system used are reported below.

**General Procedure For The In Vitro Fungicide Screen**

Assay fungi include the following plant pathogens:
Botrytis cinerea (Botry)
Pseudocerosporella herpotrichoides (Pseudo)
Pythium ultimum (Pyth)
Rhizoctonia solani (Rhiz)

The test compound (2.5-10 mg) is dissolved in 0.5 mL acetone, diluted with 4.5 mL sterile deionized water, and mixed with 95 mL of an autoclaved and partially cooled, chemically defined agar medium in a 250 mL flask. Test compounds that are insoluble are prepared as a fine suspension ultrasonically, or with a glass tissue grinder, before addition to the medium. The chemically defined medium used in these experiments is as follows:
15 g of sucrose, 0.5 g $NH_4Cl$, 0.5 g $KNO_3$, 0.5 g $KH_2PO_4$, 0.5 g $K_2HPO_4$, 0.25 g $MgSO_4.7H_2O$, 50 mg $CaCl_2.6H_2O$, 0.75 mg $FeSO_4.7H_2O$, 0.22 mg $MnSO_4.5H_2O$, 0.6 mg $CuSO_4.5H_2O$, 7.5 mg $ZnCl_2$, 2.5 mg thiamine, 60 $\mu g (NH_4)_6Mo_7O_{24}.4H_2O$, 90 $\mu g$ $H_3BO_3$, 5 $\mu g$ biotin, 20 g agar, and water to 1L.

The mixture of test compound and agar medium as described above is poured into several 100 mm x 15 mm sterile plastic plates and allowed to solidify. The surface of each plate is inoculated with a single disc of mycelium and agar cut with an 8 mm diameter cork borer from actively growing cultures of assay fungi on a chemically defined agar medium. The plates are incubated at room temperature. Growth inhibition is determined when the mycelial growth on unamended control media has reached the edge of the petri plates. The time interval varies depending on the growth rate of the fungus; two days for Pythium ultimum (Pyth) and Rhizoctonia solani (Rhiz), five days for Botrytis cinerea (Botry), and 7-10 days for Pseudocercosporella herpotrichoides (Pseudo). Each colony diameter is measured and compared with the untreated control and the percent inhibition is calculated as follows:

$$\text{\% Mycelial growth inhibition} = \frac{\text{Growth of untreated control (mm) minus the Growth of treatment (mm)}}{\text{Growth of untreated control (mm)}} \times 100$$

Each test includes a solvent blank consisting of the formulation without a test compound in addition to the untreated control.

The data obtained is shown in Table IV.

**TABLE IV**

**Evaluation of the inhibition of the mycelial growth of fungi by 2-arylpyrrole compounds**

| Compound | Rate (ppm) | % Growth Inhibition | | | |
|---|---|---|---|---|---|
| | | Pyth | Rhiz | Botry | Pseudo |
| 4,5-Dichloro-2-(p-chlorophenyl)-pyrrole-3-carbonitrile | 100 | 45.0 | 64.7 | 66.7 | 42.8 |
| | 50 | 44.7 | 73.3 | 71.4 | 40.0 |
| | 25 | 34.2 | 73.3 | 82.1 | 0 |
| 2,3-Dichloro-5-(p-chlorophenyl)-4-nitropyrrole | 100 | 92.1 | 96.0 | 96.3 | 50.0 |
| 2,3-Dichloro-4-nitro-5-(α,α,α-trifluoro-p-tolyl)-pyrrole | 100 | 100.0 | 100.0 | 100.0 | 100.0 |
| 2-Phenylpyrrole-3-carbonitrile | 100 | 100.0 | 89.0 | 95.0 | 100.0 |
| 2-(p-Chlorophenyl)pyrrole-3-carbonitrile | 100 | 95.0 | 61.0 | 68.0 | 86.0 |
| 2,3-Dichloro-4-nitro-5-phenylpyrrole | 100 | 100.0 | 81.0 | 97.0 | 100.0 |
| 2,3-Dibromo-4-nitro-5-phenylpyrrole | 100 | 97.0 | 63.0 | 87.0 | 71.0 |

**TABLE IV (Continued)**

| Compound | Rate (ppm) | % Growth Inhibition Pyth | Rhiz | Botry | Pseudo |
|---|---|---|---|---|---|
| 2-(p-Bromophenyl)-4,5-dichloro-3-nitropyrrole | 100 | 68.0 | 92.0 | 89.0 | 83.0 |
| 2-(m-Chlorophenyl)pyrrole-3-carbonitrile | 100 | 82.0 | 95.0 | 74.0 | 67.0 |
| 4,5 Dichloro-2-(o-chlorophenyl)pyrrole-3-carbonitrile | 100 | 0 | 52.6 | 3.1 | 16.7 |
| 2,5-dibromo-3-nitro-4-phenylpyrrole | 10 | 100 | 100 | | 100 |
| | 1 | 100 | | | |
| 4-bromo-2-(p-chlorophenyl)-5-(trifluoromethyl)pyrrole-3-carbonitrile | 10 | | 100 | | |
| 5-(3,4-dichlorophenyl)-4-nitropyrrole-2-carbonitrile | 10 | 100 | 100 | | |
| | 1 | 100 | | | |

TABLE IV (Continued)

| Compound | Rate (ppm) | % Growth Inhibition Pyth | Rhiz | Botry | Pseudo |
|---|---|---|---|---|---|
| 3-nitro-2-phenyl-4,5-bis (trifluoromethyl) pyrrole | 10 | 100 | 100 | | |
| 2,5-dichloro-3-nitro-4-phenyl-pyrrole | 10<br>1 | 100<br>100 | 100 | | |
| 2,5-dichloro-4-(p-chlorophenyl) pyrrole-3-carbonitrile | 10 | 100 | | | |
| 5-(p-chlorophenyl) pyrrole-2,4-dicarbonitrile | 10 | 100 | | | |
| 5-[p-(trifluoro-methoxy)phenyl] pyrrole-2,3-dicarbonitrile | 10<br>1 | 100<br>100 | | | |
| 3-bromo-5-(3,4-dichlorophenyl)-4-nitropyrrole-2-carbonitrile | 10<br>1 | 100<br>100 | | | |

TABLE IV (Continued)

| Compound | Rate (ppm) | % Growth Inhibition | | | |
|---|---|---|---|---|---|
| | | Pyth | Rhiz | Botry | Pseudo |
| 5-(trifluoromethyl)-2-(α,α,α-trifluoro-p-tolyl)pyrrole-3-carbonitrile | 10 | 100 | | | |
| | 1 | 100 | | | |

## Claims

1. A method for the control of plant pathogenic fungi comprising contacting said fungi with a fungicidally effective amount of a compound having the structure:

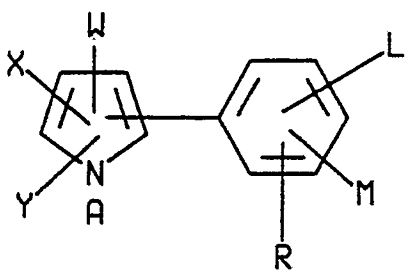

wherein X is H, F, C1, Br, I or $CF_3$; Y is H, F, C1, Br, I, $CF_3$ or CN; W is CN or $NO_2$ and A is H; $C_1$-$C_4$ alkyl optionally substituted with from one to three halogen atoms, one hydroxy, one $C_1$-$C_4$ alkoxy or one $C_1$-$C_4$ alkylthio, one phenyl optionally substituted with $C_1$-$C_3$ alkyl or $C_1$-$C_3$ alkoxy or with one to three halogen atoms, one phenoxy optionally substituted with one to three halogen atoms or one benzyloxy optionally substituted with one halogen substituent; $C_1$-$C_4$ carbalkoxymethyl; $C_3$-$C_4$ alkenyl optionally substituted with from one to three halogen atoms; cyano; $C_3$-$C_4$ alkynyl optionally substituted with one halogen atom; di($C_1$-$C_4$ alkyl) aminocarbonyl; or $C_4$-$C_6$ cycloalkylaminocarbonyl; L is H, F, C1 or Br; and M and R are each independently H, $C_1$-$C_3$ alkyl, $C_1$-$C_3$ alkoxy, $C_1$-$C_3$ alkythio, $C_1$-$C_3$ alkysulfinyl, $C_1$-$C_3$ alkysulfonyl, cyano, F, C1, Br, I, nitro, $CF_3$, $R_1CF_2Z$, $R_2CO$ or $NR_3R_4$, and when M and R are on adjacent positions then with the carbon atoms to which they are attached they may form a ring in which MR represents the structure:

$-OCH_2O-$, $-OCF_2O-$ or 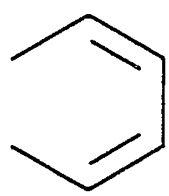 ;

Z is S(O)n or O; $R_1$ is H, F, $CHF_2$, CHFC1, or $CF_3$; $R_2$ is $C_1$-$C_3$ alkyl, $C_1$-$C_3$ alkoxy, or $NR_3R_4$; $R_3$ is H or $C_1$-$C_3$ alkyl; $R_4$ is H, $C_1$-$C_3$ alkyl, or $R_5CO$; $R_5$ is H or $C_1$-$C_3$ alkyl; and n is an integer of 0, 1 or 2, provided that when either X or Y or both are H, then

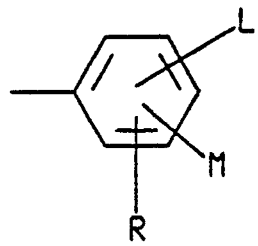

must be fixed to one of the two positions on the pyrrole ring adjacent to the nitrogen atom; provided also that when W is cyano and X and Y are both H, then at least one of L, M, and R is other than H; and further provided that when W is $NO_2$, A is $C_1$-$C_4$ alkyl, and either X or Y is Cl, then neither M nor R is $C_1$-$C_3$ alkoxy.

2. A method according to claim 1 wherein the plant pathogenic fungi are the causative agents for cucumber downy mildew, tomato late blight, rice blast, wheat leaf rust, apple scab, grape downy mildew, wheat powdery mildew or soilborne diseases.

3. A method according to claim 1 wherein the compound has the formula:

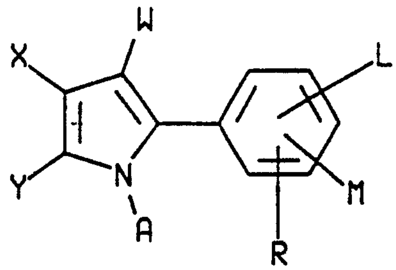

4. A method to protect agronomic plants from attack by plant pathogenic fungi which are causative agents for cucumber downy mildew, tomato late blight, apple scab, grape downy mildew, wheat powdery mildew, rice blast, or wheat leaf rust comprising applying to said plants a fungicidally effective amount of a compound having the structure:

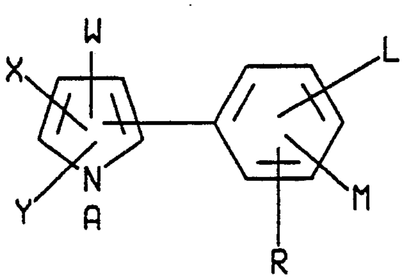

wherein X is H, F, C1, Br, I or $CF_3$; Y is H, F, C1, Br, I, $CF_3$ or CN; W is CN or $NO_2$ and A is H; $C_1$-$C_4$ alkyl optionally substituted with from one to three halogen atoms, one hydroxy, one $C_1$-$C_4$ alkoxy or one $C_1$-$C_4$ alkylthio, one phenyl optionally substituted with $C_1$-$C_3$ alkyl or $C_1$-$C_3$ alkoxy or with one to three halogen atoms, one phenoxy optionally substituted with one to three halogen atoms or one benzyloxy optionally substituted with one halogen substitutent; $C_1$-$C_4$ carbalkoxymethyl; $C_3$-$C_4$ alkenyl optionally substituted with from one to three halogen atoms; cyano; $C_3$-$C_4$ alkynyl optionally substituted with one halogen atom; di-($C_1$-$C_4$ alkyl) aminocarbonyl; or $C_4$-$C_6$ cycloalkylaminocarbonyl; L is H, F, C1 or Br; and M and R are each independently H, $C_1$-$C_3$ alkyl, $C_1$-$C_3$ alkoxy, $C_1$-$C_3$ alkythio, $C_1$-$C_3$ alkysulfinyl, $C_1$-$C_3$ alkysulfonyl, cyano, F, C1, Br, I, nitro, $CF_3$, $R_1$,$CF_2Z$, $R_2CO$ or $NR_3R_4$, and when M and R are on adjacent positions then with the carbon atoms to which they are attached they may form a ring in which MR represents the structure:

$-OCH_2O-$, $-OCF_2O-$ or 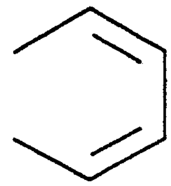 ;

Z is S(O)n or O; $R_1$ is H, F, $CHF_2$, CHFC1, or $CF_3$; $R_2$ is $C_1$-$C_3$ alkyl, $C_1$-$C_3$ alkoxy, or $NR_3R_4$; $R_3$ is H or $C_1$-$C_3$ alkyl; $R_4$ is H, $C_1$-$C_3$ alkyl, or $R_5CO$; $R_5$ is H or $C_1$-$C_3$ alkyl; and n is an integer of 0, 1 or 2, provided that when either X or Y or both are H, then

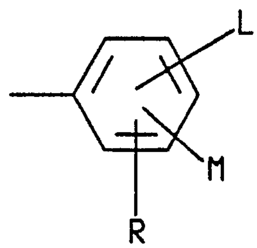

must be fixed to one of the two positions on the pyrrole ring adjacent to the nitrogen atom; provided also that when W is cyano and X and Y are both H, then at least one of L, M, and R is other than H; and further provided that when W is $NO_2$, A is $C_1$-$C_4$ alkyl, and either X or Y is Cl, then neither M nor R is $C_1$-$C_3$ alkoxy.

5. A method according to claim 4 wherein the compound has the formula:

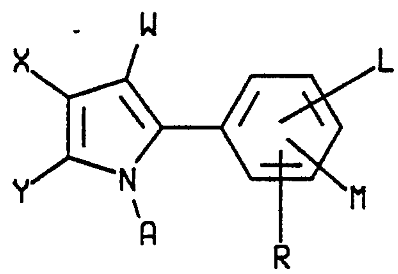

6. A method to protect agronomic plants from attack by plant pathogenic fungi which are causative agents for cucumber downy mildew, tomato late blight, apple scab, grape downey mildew, wheat powdery mildew, rice blast, or wheat leaf rust comprising applying to soil surrounding said plants a fungicidally effective amount of a compound having the structure:

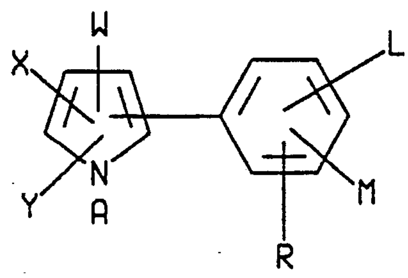

wherein X is H, F, C1, Br, I or $CF_3$; Y is H, F, C1, Br, I, $CF_3$ or CN; W is CN or $NO_2$ and A is H; $C_1$-$C_4$ alkyl optionally substituted with from one to three halogen atoms, one hydroxy, one $C_1$-$C_4$ alkoxy or one $C_1$-$C_4$ alkylthio, one phenyl optionally substituted with $C_1$-$C_3$ alkyl or $C_1$-$C_3$ alkoxy or with one to three halogen atoms, one phenoxy optionally substituted with one to three halogen atoms or one benzyloxy optionally substituted with one halogen substitutent; $C_1$-$C_4$ carbalkoxymethyl; $C_3$-$C_4$ alkenyl optionally substituted with

from one to three halogen atoms; cyano; $C_3$-$C_4$ alkynyl optionally substituted with one halogen atom; di-($C_1$-$C_4$ alkyl) aminocarbonyl; or $C_4$-$C_6$ cycloalkylaminocarbonyl; L is H, F, C1 or Br; and M and R are each independently H, $C_1$-$C_3$ alkyl, $C_1$-$C_3$ alkoxy, $C_1$-$C_3$ alkoxy, $C_1$-$C_3$ alkythio, $C_1$-$C_3$ alkysulfinyl, $C_1$-$C_3$ alkysulfonyl, cyano, F, C1, Br, I, nitro, $CF_3$, $R_1CF_2Z$, $R_2CO$ or $NR_3R_4$, and when M and R are on adjacent positions then with the carbon atoms to which they are attached they may form a ring in which MR represents the structure:

$-OCH_2O-$, $-OCF_2O-$ or 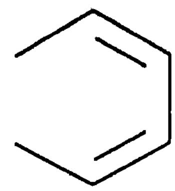 ;

Z is S(O)n or O; $R_1$ is H, F, $CHF_2$, CHFC1, or $CF_3$; $R_2$ is $C_1$-$C_3$ alkyl, $C_1$-$C_3$ alkoxy, or $NR_3R_4$; $R_3$ is H or $C_1$-$C_3$ alkyl; $R_4$ is H, $C_1$-$C_3$ alkyl, or $R_5CO$; $R_5$ is H or $C_1$-$C_3$ alkyl; and n is an integer of 0, 1 or 2, provided that when either X or Y or both are H, then

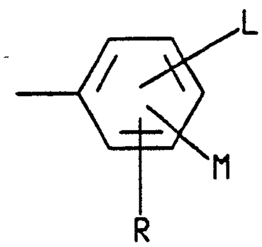

must be fixed to one of the two positions on the pyrrole ring adjacent to the nitrogen atom; provided also that when W is cyano and X and Y are both H, then at least one of L, M, and R is other than H; and further provided that when W is $NO_2$, A is $C_1$-$C_4$ alkyl, and either X or Y is Cl, then neither M nor R is $C_1$-$C_3$ alkoxy.

7. A method according to claim 6 wherein the compound has the formula:

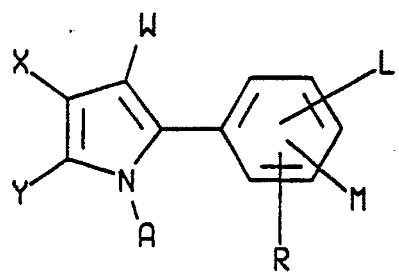

8. A fungicidal composition comprising a fungicidally effective amount of a compound having the structure:

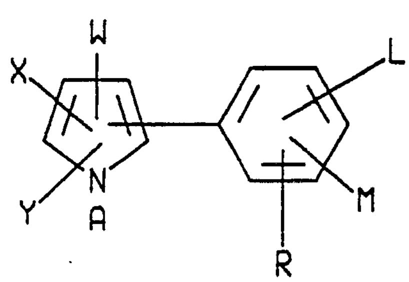

wherein X is H, F, C1, Br, I or $CF_3$; Y is H, F, C1, Br, I, $CF_3$ or CN; W is CN or $NO_2$ and A is H; $C_1$-$C_4$ alkyl optionally substituted with from one to three halogen atoms, one hydroxy, one $C_1$-$C_4$ alkoxy or one $C_1$-$C_4$ alkylthio, one phenyl optionally substituted with $C_1$-$C_3$ alkyl or $C_1$-$C_3$ alkoxy or with one to three halogen atoms, one phenoxy optionally substituted with one to three halogen atoms or one benzyloxy optionally substituted with one halogen substituent; $C_1$-$C_4$ carbalkoxymethyl; $C_3$-$C_4$ alkenyl optionally substituted with from one to three halogen atoms; cyano; $C_3$-$C_4$ alkynyl optionally substituted with one halogen atom; di-($C_1$-$C_4$ alkyl) aminocarbonyl; or $C_4$-$C_6$ cycloalkylaminocarbonyl; L is H, F, C1 or Br; and M and R are each independently H, $C_1$-$C_3$ alkyl, $C_1$-$C_3$ alkoxy, $C_1$-$C_3$ alkythio, $C_1$-$C_3$ alkysulfinyl, $C_1$-$C_3$ alkysulfonyl, cyano, F, C1, Br, I, nitro, $CF_3$, $R_1CF_2Z$, $R_2CO$ or $NR_3R_4$, and when M and R are on adjacent positions then with the carbon atoms to which they are attached they may form a ring in which MR represents the structure:

$-OCH_2O-$, $-OCF_2O-$ or 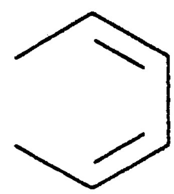 ;

Z is S(O)n or O; $R_1$ is H, F, $CHF_2$, CHFC1, or $CF_3$; $R_2$ is $C_1$-$C_3$ alkyl, $C_1$-$C_3$ alkoxy, or $NR_3R_4$; $R_3$ is H or $C_1$-$C_3$ alkyl; $R_4$ is H, $C_1$-$C_3$ alkyl, or $R_5CO$; $R_5$ is H or $C_1$-$C_3$ alkyl; and n is an integer of 0, 1 or 2, provided that when either X or Y or both are H, then

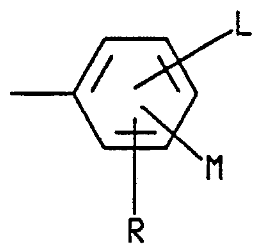

must be fixed to one of the two positions on the pyrrole ring adjacent to the nitrogen atom; provided also that when W is cyano and X and Y are both H, then at least one of L, M, and R is other than H; and further provided that when W os $NO_2$, A is $C_1$-$C_4$ alkyl, and either X or Y is Cl, then neither M nor R is $C_1$-$C_3$ alkoxy.

9. A composition according to claim 8 wherein the compound has the formula:

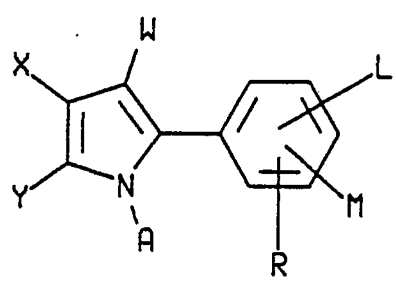

10. A composition according to claim 8 wherein the compound is formulated as a liquid spray, a fine ultrasonically prepared suspension, or as a dust or granular formulation.